(19) Europäisches Patentamt
European Patent Office
Office européen des brevets

(11) **EP 3 285 759 B1**

(12) **EUROPEAN PATENT SPECIFICATION**

(45) Date of publication and mention
of the grant of the patent:
**13.11.2019 Bulletin 2019/46**

(21) Application number: **16717670.0**

(22) Date of filing: **22.04.2016**

(51) Int Cl.:
*A61K 31/30* (2006.01)    *A61K 9/00* (2006.01)
*A61K 47/10* (2017.01)    *A61K 9/06* (2006.01)
*A61K 47/38* (2006.01)    *A61K 9/14* (2006.01)
*A61K 33/34* (2006.01)    *A61P 31/04* (2006.01)

(86) International application number:
**PCT/EP2016/059074**

(87) International publication number:
**WO 2016/170152 (27.10.2016 Gazette 2016/43)**

(54) **ANTIBACTERIAL COMPOSITIONS COMPRISING COPPER OXO-HYDROXIDE NANOPARTICLES AND THEIR PREPARATION**

KUPFER OXO-HYDROXID NANOPARTIKEL ENTHALTENDE ANTIBAKTERIELLE ZUSAMMENSETZUNGEN UND DEREN VORBEREITUNG

COMPOSITIONS PHARMACEUTIQUES COMPRENANT DES NANOPARTICULES DE CUIVRE OXO-HYDROXYDE ET LEUR PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2015 GB 201507002**

(43) Date of publication of application:
**28.02.2018 Bulletin 2018/09**

(73) Proprietor: **United Kingdom Research and Innovation
Swindon SN2 1FL (GB)**

(72) Inventors:
• **BASTOS, Carlos André Passos
Cambridge
Cambridgeshire CB1 3RY (GB)**
• **BRUGGRABER, Sylvaine Francoise Aline Comberton
Cambridgeshire CB23 7DD (GB)**
• **FARIA, Nuno, Jorge, Rodrigues Milton Ernest
Bedfordshire MK44 1RX (GB)**
• **POWELL, Jonathan, Joseph Cambridge
Cambridgeshire CB4 3JD (GB)**

(74) Representative: **Mewburn Ellis LLP
City Tower
40 Basinghall Street
London EC2V 5DE (GB)**

(56) References cited:
WO-A1-2008/096130    WO-A1-2012/101407
WO-A1-2015/121666    US-A1- 2008 147 019

**Description**

**Field of the Invention**

The present invention relates to antibacterial compositions for use in the treatment of wounds or the treatment or prevention of microbial infection, wherein the composition comprises ligand-modified copper oxo-hydroxide nanoparticles.

**Background of the Invention**

Development of new antimicrobials has progressively slowed down since the 1980s, leaving a bleak scenario in the face of emerging multi-drug resistant pathogens. So called 'superbugs' are increasingly recognised as a global threat to public health, driving exploration of new antimicrobials - including inorganic agents, such as those based on copper and silver. These metals have had historical usage and, significantly, are hypothesised to act via a multiplicity of biocidal mechanisms - which could potentially enhance clinical longevity by requiring microorganisms to undergo multiple mutations to gain resistance. Of the two metals, silver has shown greater antimicrobial efficacy: however, cost, *in vivo* toxicity and chemical instability are likely to limit its utility for clinical applications such as the healing of infected wounds. Copper, whilst less efficacious, is inexpensive and, being an essential micronutrient, is better tolerated by man, allowing greater doses to be used. However, owing to its lower biocidal efficacy, the development of delivery systems which maximise bioavailability of the free copper is critical to its use in clinical settings.

GB 1600449 (Mooney Chemicals Inc.) relates to resin or soap-like substances in which crystalline metal oxide particles are surrounded in an amorphous matrix of organic molecules in a stoichiometric manner to produce metal oxide compositions that can be dissolved in non-polar (oil-like) solvents, mainly for use in catalysis. GB 1600449 demonstrates that these compositions retain unmodified crystallite cores by X ray diffraction that shows that the organic molecules are coated on the surface of the particles rather than being incorporated inside them.

WO 2008/096130 (Medical Research Council) describes ligand-modified poly oxo-hydroxy metal ion materials and their uses are disclosed, in particular for nutritional, medical, cosmetic or biologically related applications such as the treatment of a deficiency related to a component of the material such as anaemia or for the removal of an endogenous substance capable of binding to the material. Examples of these types of materials for use as phosphate binding materials are described in WO 2010/015827.

WO 2012/101407 relates to oxygen sensors for use in product packaging for storing an article in a packaging envelope under modified atmosphere conditions, in which the oxygen sensors are based on metal oxo-hydroxides that are optionally modified with one or more ligands. The sensors may be present in a hydrated, oxygen permeable matrix, for example formed from a material, such as gelatine.

DE 20205014332 relates to organometallic nanopowders containing chemically reactive groups and their use in the formation of polymeric composites.

US 2008/0147019 describes the use of antibacterial composition in which metallic silver or copper is incorporated into a chitosan matrix to protect the metal nanoparticles from uncontrolled oxidation.

The development of approaches for the effective delivery of antimicrobial metal ions such as copper, remains an unresolved problem in the art, especially for use in a clinical setting.

**Summary of the Invention**

Broadly, the present invention relates to nanoparticles formed from copper oxo-hydroxide that are capable of delivering biocidal concentrations of copper, typically in the form of free copper ions ($Cu^{2+}$). The nanoparticle compositions of the present invention achieve this result by providing small particles, typically having mean diameters in the range of 1 nm to 100 nm, and more preferably in the range of 1 nm to 10 nm, having comparatively high surface area-to-volume ratio and enhanced reactivity compared to the corresponding bulk counterpart materials and which are sufficiently labile to release the free copper efficiently, enabling them to act as pharmaceutical or antibacterial compositions, unlike prior art copper nanoparticles. In preferred embodiments, this is achieved by through ligand modification of the copper oxo-hydroxide in which one or more ligands are non-stoichiometrically substituted for the oxo or hydroxy groups of the copper oxo-hydroxide. The experiments described herein demonstrate that the copper oxo-hydroxide nanoparticles are as effective as antibacterial agents and are superior compared to commercially available copper oxide (CuO) nanoparticles, silicate stabilised copper hydroxide nanoparticles and copper complexes with strong chelating agents, such as EDTA. The copper oxo-hydroxide nanoparticle compositions of the present invention are preferably modified with carboxylic acid ligands, or ionised forms thereof, such as tartrate and adipate.

Accordingly, in a first aspect, the present invention provides an antibacterial composition for use in the treatment of wounds, wherein the composition comprises ligand-modified copper oxo-hydroxide nanoparticles and a pharmaceutically acceptable carrier, wherein the copper oxo-hydroxide nanoparticles have a structure in which the one or more ligands are non-stoichiometrically substituted for the oxo or hydroxy groups, wherein the one or more ligands comprise a carboxylic acid ligand, or an ionised form thereof and the copper is present in pharmaceutical formulation as free copper ions ($Cu^{2+}$).

As the copper oxo-hydroxide nanoparticles have a polymeric structure in which the ligands are distributed within the solid phase structure of the copper oxo-hydroxide, rather than simply being coated or physically adsorbed on the surface of the particles of copper oxo-hydroxide, the present inventors believe that the inclusion of the ligands helps to modulate the dissolution of the nanoparticles to provide free soluble copper ions available for biocidal use. It is preferred that the copper oxo-hydroxide nanoparticles have one or more reproducible physico-chemical properties, for example dissolution profile, percentage of soluble copper made available as a function of total copper present in the nanoparticles and/or biocidal activity of the nanoparticles in a bacterial growth inhibition assay and/or retention of lability upon resuspending a composition that has been dried.

In a further aspect, the present invention provides an antibacterial composition for use in the treatment or prevention of a microbial infection, wherein the composition comprises ligand-modified copper oxo-hydroxide nanoparticles and a pharmaceutically acceptable carrier, wherein the copper oxo-hydroxide nanoparticles have a structure in which the one or more ligands are non-stoichiometrically substituted for the oxo or hydroxy groups, wherein the one or more ligands comprise a carboxylic acid ligand, or an ionised form thereof and the copper is present in pharmaceutical formulation as free copper ions ($Cu^{2+}$).

In a further aspect, the present invention provides a process for producing a copper oxo-hydroxide nanoparticle composition according to the present invention, the process comprising:

(a) mixing the solution comprising $Cu^{2+}$ and a carboxylic acid ligand, and optionally one or more further ligands or reaction components, in a reaction medium at a first pH(A) at which the components are soluble;
(b) changing the pH(A) to a second pH(B) to cause a solid precipitate or a colloid of the copper oxo-hydroxide nanoparticle composition to be formed;
(c) separating, and optionally drying and/or formulating, the copper oxo-hydroxide nanoparticle composition produced in step (b).

Also described is a composition comprising ligand-modified copper oxo-hydroxide nanoparticles, wherein the copper oxo-hydroxide nanoparticles have a structure in which the one or more ligands are non-stoichiometrically substituted for the oxo or hydroxy groups, wherein the one or more ligands comprise a carboxylic acid ligand, or an ionised form thereof, as obtainable by the above process.

Also described is an article having a surface treated to include ligand-modified copper oxo-hydroxide nanoparticles of the present invention, wherein the nanoparticles provide the surface of the article with antibacterial properties. Examples of articles treatable in this way, such as medical equipment, bandages and dressings, are provided below.

It will be understood that the coated substrates may be for use in a method of medical treatment, for example for the treatment and/or prophylaxis of microbial infection or the treatment of wounds. The substrate may also be useful for the treatment and/or prophylaxis of skin disorders or disorders of mucous membranes.

The present invention also provides use of a ligand modified copper oxo-hydroxide nanoparticle composition in the manufacture of a medicament for the treatment and/or prophylaxis of skin disorders or disorders of mucous membranes. It is understood that the medicament may be a coating or coated substrate of the present invention, for example a coated wound dressing, or a coated medical device such as an implantable medical device, for example a stent.

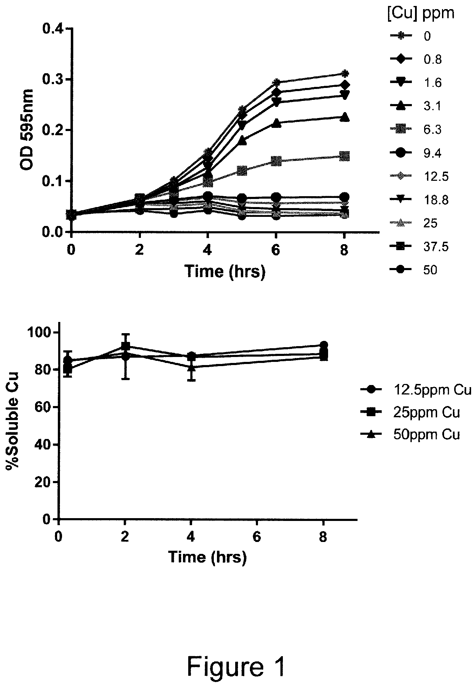

Embodiments of the present invention will now be described by way of example and not limitation with reference to the accompanying figures and examples. Figures 1-6 are provided for comparative purposes.

## Brief Description of the Figures

**Figure 1.** Growth curves of *E. coli* with exposure to copper chloride at 0 to 50ppm Cu. Bacterial growth was followed through the measurement of optical density (OD) over time (top). Copper chloride solubility in the bacterial growth medium over the period of the assay (bottom). Error bars represent standard deviations (n=3).

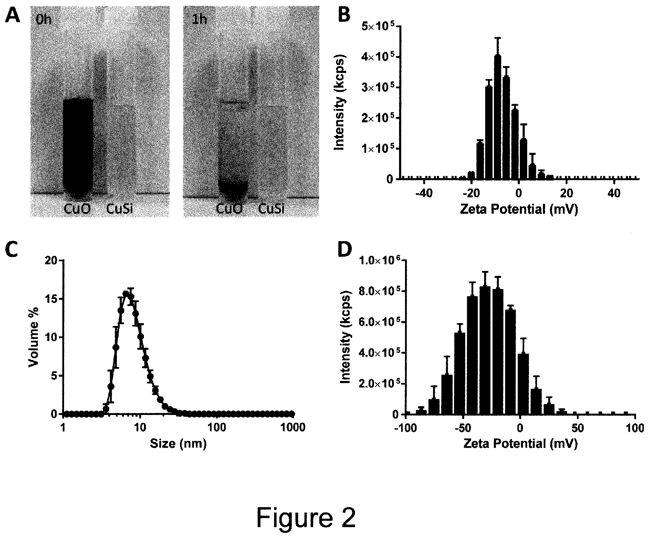

**Figure 2. (A)** CuO and CuSi NPs (as per experimental examples) in water at ca. 1270ppm, immediately after being dispersed (0h) and one hour after standing without agitation, showing the formation of large CuO agglomerates (black sediment), unlike CuSiNPs that remained stable in suspension. **(B)** Zeta potential of CuO NPs in a water suspension at ca. 1270ppm Cu. Particle size distribution **(C)** and zeta potential **(D)** of CuSiNPs, both analysed at pH 12 in water at *ca.* 1200ppm copper. Error bars represent standard deviations (n=3).

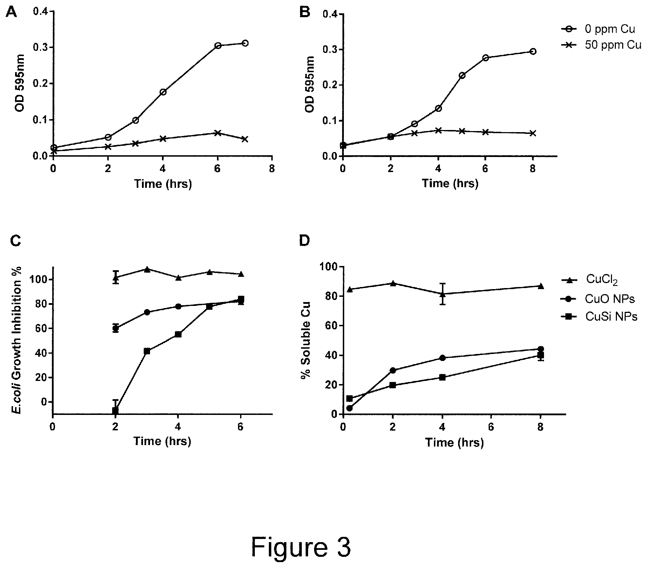

**Figure 3.** *E. coli* growth curves after exposure to CuO NPs (A) and CuSi NPs at 50ppm Cu (B), and their respective bacterial growth inhibition in comparison to $CuCl_2$, soluble copper control (C). Dissolution of CuSi NPS and CuO NPs in bacterial culture medium, at 50ppm Cu (D). Error bars represent standard deviations (n=3).

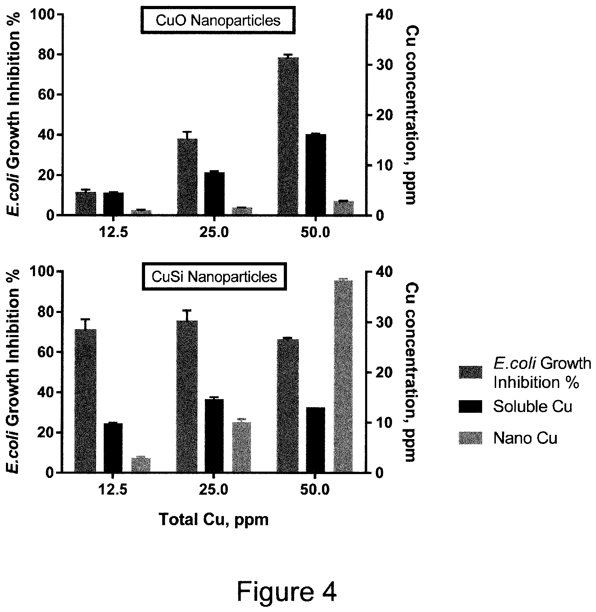

**Figure 4.** Comparison of *E. coli* growth inhibition with levels of nanoparticulate and soluble copper in the bacterial culture medium, at 3 different concentrations of copper (12.5, 25 and 50 ppm Cu) after 4 hours of incubation with CuO NPs (Top) and CuSi NPs (bottom).

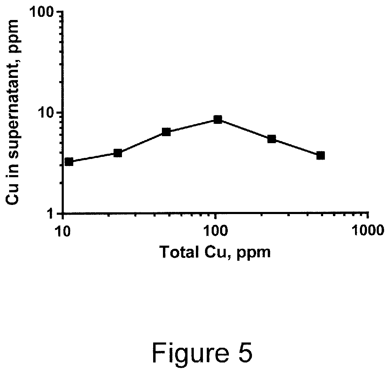

**Figure 5.** Dispersible Cu in a MOPS buffer at pH $7.4\pm0.2$ upon dilution of $CuCl_2$ to a range of concentrations from 10 to 500ppm (n=3).

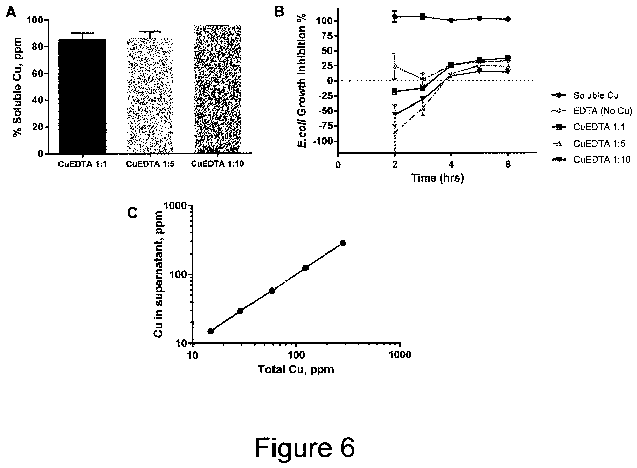

**Figure 6.** (A) Solubility of Cu-EDTA stocks at pH $7.5 \pm 0.2$ in water at ca. 1270ppm Cu. Error bars represent standard deviations of two analytical replicates. (B) *E. coli* growth inhibition after incubation with CuEDTA complexes at different ratios. Note that negative values represent an increase in initial growth compared to copper-free cultures. (C) Dispersible copper in a MOPS buffer at pH $7.4\pm0.2$ upon dilution of CuSi nanoparticles to a range of concentrations from 10 to 500ppm. Error bars represent standard deviations (n=3).

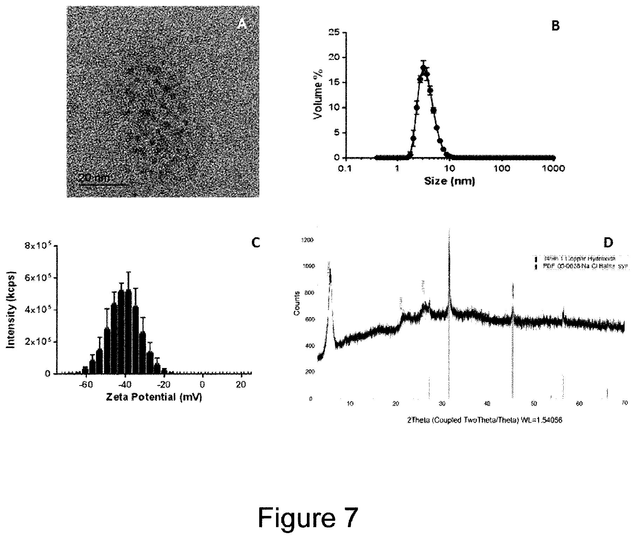

**Figure 7.** Characterisation of CuTartAd nanoparticles (prepared as per experimental examples). A - TEM analysis of a suspension, as prepared, at pH 8 containing ca. 2500ppm Cu (suspension was dropcast on a TEM grid). B - Hydrodynamic particle size distribution of the same nanoparticles analysed by Dynamic Light Scattering showing a mean size of $3.72 \pm 0.04$nm. C. Zeta Potential of CuTartAd NPs at pH 8, ca. 1000ppm Cu. D. XRD spectrum of amorphous CuTartAd nanoparticles. Peaks in red correspond to halite (crystalline NaCl), which was formed by neutralisation of an acidic chloride-containing solution with sodium hydroxide. Error bars represent standard deviations of three analytical replicates.

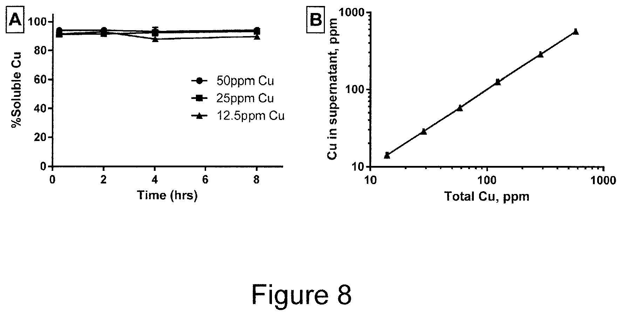

**Figure 8.** A) Dissolution profile of CuTartAd NPs in bacterial culture medium at 12.5, 25 and 50 ppm Cu; Error bars represent standard deviations (n=3). B) Dispersible copper in MOPS buffer at pH $7.4 \pm 0.2$ upon dilution of CuTartAd NPs to a range of concentrations from 10 to 500ppm. Error bars represent standard deviations (n=3).

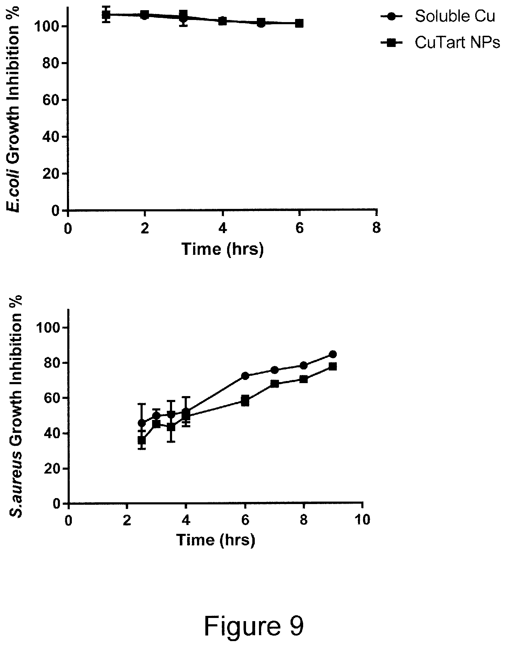

**Figure 9.** *E. coli* (top) and *S. aureus* (bottom) growth inhibition after incubation with soluble Cu and CuTartAd nanoparticles both at 50ppm Cu. Error bars represent standard deviations (n=2).

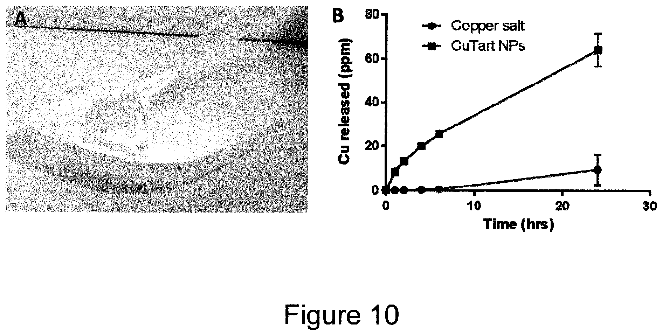

**Figure 10.** HEC matrix containing CuTartAd NPs at 250ppm Cu (A). Release of Cu from HEC matrices containing 250ppm Cu. Error bars represent standard deviations (n=3). This assay consisted of exposing the copper-containing HEC - with specific surface area ($7.1$ cm$^2$) - to a bicarbonate buffered solution at pH $7.0\pm0.2$, following copper concentration over time.

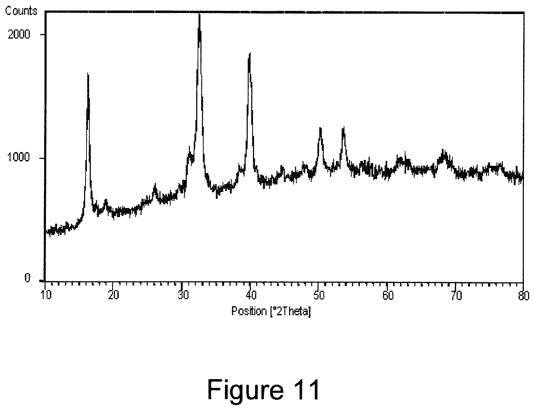

**Figure 11.** XRD spectrum of the unmodified copper hydroxide synthesized for comparative purposes (Example 4.N6) was also obtained (bottom). The latter showed a crystalline pattern corresponding to paratacamite, a copper hydroxide of chemical formula $Cu_2(OH)_3Cl$ in which a chlorine atom was incorporated in the mineral structure (bottom).

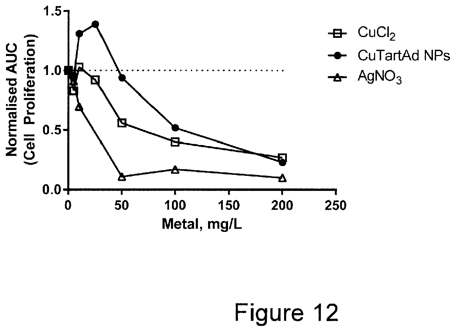

**Figure 12.** Cell proliferation of skin fibroblasts (cell line CCD-25Sk) upon exposure to $CuCl_2$, $AgNO_3$ and tartrate adipate modified copper oxo-hydroxide nanoparticles (CuTartAd NPs) for 48 hours.

## Detailed Description

*Copper oxo-hydroxide nanoparticles compositions*

The production and characterisation of solid ligand-modified poly oxo-hydroxy metal ion materials, and in particular materials based on ferric iron oxo-hydroxide, are described in our earlier applications WO 2008/096130 and WO 2010/015827. Corresponding processes were adapted in the work reported in the present application to provide the ligand modified copper oxo-hydroxide nanoparticle compositions of the present invention that have uses in antibacterial and antimicrobial applications, for example for promoting wound healing.

In general, this class of materials may be represented by the non-stoichiometric formula $(M_xL_y(OH)_n)$, where M represents one or more metal ions, L represents one or more ligands and OH represents oxo or hydroxy groups, depending on whether the groups are bridging (oxo groups) or surface groups in the solid oxo-hydroxide material. As is well known in the art, non-stoichiometric compounds are chemical compounds with an elemental composition that cannot be represented by a ratio of well-defined natural numbers, i.e. the x, y and n subscripts in the formula above will not necessarily all be natural numbers, even though the materials can be made in a reproducible manner and have consistently reproducible properties. Preferably, the ligand modified copper oxo-hydroxides of the present invention have a polymeric structure in which the ligands are substantially randomly substituted for the oxo or hydroxy groups. This provides copper oxo-hydroxide nanoparticles having one or more reproducible physicochemical properties, for example compositions having one or more of a mean particle size diameter in the range of about 1 nm to about 100 nm (for example as determined by dynamic light scattering, see section 1.2.1), a reproducible dissolution profile, compositions in which the nanoparticles are substantially amorphous (for example as determined using X-ray diffraction or transmission electron microscopy, see sections 1.2.3 and 1.2.4) and/or compositions in which the nanoparticles have demonstrable metal-ligand bonding (for example as determined using infra-red spectroscopy). Additionally or alternatively, the copper oxo-hydroxide nanoparticle compositions are capable of releasing a percentage of soluble copper that is preferably at least 25% of the total copper present in the composition, more preferably at least 30%, more preferably at least 40% and most preferably at least 50%. The release of soluble copper may be measured in a free copper release assay (e.g. as described in the examples below). The biocidal properties of the copper oxo-hydroxide nanoparticle compositions may be measured using a bacterial growth inhibition assay and preferably achieves at least 50% bacterial growth inhibition, more preferably at least 60% bacterial growth inhibition, more preferably at least 70% bacterial growth inhibition, and more preferably at least 90% bacterial growth inhibition under standardised conditions. In a preferred embodiment, full (100%) inhibition of *E. coli* growth is achieved using the antimicrobial compositions of the present invention, for example in an assay in which *E. coli* was exposed to the ligand modified copper oxo-hydroxide nanoparticles for 6 hours with copper concentrations above 25mg/L fully inhibiting (100%) *E. coli* growth in these specific conditions. A further example of a suitable growth inhibition assay is provided in section 1.3.2.

Typically, the metal ion (e.g. $Cu^{2+}$) will originally be present in the form of a salt that in the preparation of the materials may be dissolved and then induced to form poly oxo-hydroxy co-complexes with ligand (L). As described below, other metal ions may be present in addition to copper ions ($Cu^{2+}$). While not wishing to be bound by any particular theory, the present inventors believe that in these materials, and in the ligand modified copper oxo-hydroxide nanoparticles of the present invention, some of the ligand used to modify the metal oxo-hydroxide is integrated within the solid phase through formal M-L bonding, i.e. not all of the ligand (L) is simply trapped or adsorbed in the bulk material and/or is adsorbed or coated on the surface of the particles of the metal oxo-hydroxide material. The bonding of the metal ion in the materials can be determined using physical analytical techniques such as infrared spectroscopy where the spectra will have peaks characteristic of the bonds between the metal ion and the ligand (L), as well as peaks characteristic of other bonds present in the material such as M-O, O-H and bonds in the ligand species (L). Alternatively or additionally, the ligand species may be introduced into the solid phase structure by the substitution of oxo or hydroxyl groups by ligand molecules in a manner that decreases overall order in the solid phase material, so that the materials have a more amorphous nature compared, for example, to the structure of the corresponding unmodified copper hydroxide. The presence of a more disordered or amorphous structure can readily be determined by the skilled person using techniques well known in the art. One exemplary technique is Transmission Electron Microscopy (TEM). High resolution transmission electron microscopy allows the crystalline pattern of the material to be visually assessed. It can indicate the primary particle size and structure (such as d-spacing), give some information on the distribution between amorphous and crystalline material. This may be especially apparent using high angle annular dark field aberration-corrected scanning transmission electron microscopy due to the high contrast achieved while maintaining the resolution, thus allowing the surface as well as the bulk of the primary particles of the material to be visualised.

The copper oxo-hydroxide nanoparticles disclosed herein use copper ions ($Cu^{2+}$) to provide compositions that are capable of delivering biologically effective concentrations of biocidal copper, for example for use as an antibacterial or antimicrobial agents. The compositions of the present invention may further have the advantage of being biologically compatible and non toxic in view of the general physiological tolerance to copper.

By way of background, it is well known in the art that copper oxides, hydroxides and oxo-hydroxides are composed of $Cu^{2+}$ together with 0 and/or OH and are collectively referred to in this patent and known in the art as "copper oxo-hydroxides". In addition to the presence of copper ions ($Cu^{2+}$), other metal ions may be present such as metal cations selected from $Ca^{2+}$, $Mg^{2+}$, $Ag^+$, $Al^{3+}$, $Fe^{3+}$ and/or $Zn^{2+}$. In particular, it may be desirable to include further metal cations with antimicrobial properties, such as $Ag^+$. A further preferred type of materials include $Zn^{2+}$, in addition to copper ions.

The copper oxo-hydroxide nanoparticles of the present invention are based on the development of compositions designed for optimal delivery of soluble copper, for example for use in applications where antibacterial activity of soluble copper is desirable. The comparative examples herein show that when dispersed at the concentrations that are required in clinical formulations, common copper salts tend to be precipitated as large, and biocidally inactive, copper hydroxides (as shown in Figure 5). Moreover, while the addition of complexing agents (e.g. EDTA) prevents the formation of such agglomerates, and is capable of keeping copper in solution, these preparations showed modest inhibition of bacterial growth due to the limited bioavailability of complexed copper ions. These experiments showed that despite copper ions being the active form responsible to biocidal activity that copper salts are not a good way of delivering them as the salt forms tend to convert to insoluble copper hydroxides. The present inventors realised that both of these approaches are undesirable as the copper ions are biologically unavailable, either by being present in agglomerates or by being strongly complexed by agents, such as EDTA.

Accordingly, the present invention concerns nanoparticulate systems for the delivery of free copper ions by functionalising copper oxo-hydroxide nanoparticles with ligands, for example dietary ligands such as carboxylic acids or amino acids. In a preferred approach, the mineral phase of copper oxo-hydroxide nanoparticles was modified through the use of carboxylate ligands, such as tartrate, gluconate, adipate and/or glutathione, which conferred negative surface charge, and stabilised the nanoparticles in aqueous environments.

Preferably, the copper oxo-hydroxide nanoparticles of the present invention have mean diameter ranges 1 to 100 nm, 1 to 50 nm, 1 to 20 nm, 1 to 10nm. The size of the particles of copper oxo-hydroxide nanoparticles can be determined using techniques well known in the art such as dynamic light scattering, as demonstrated in the examples in section 1.2.1. By way of example, this may be carried out using a Zetasizer NanoZS (Malvern Instruments). In a typical experiment, 0.5 to 1 ml of a suspension of copper oxo-hydroxide nanoparticles may be transferred into a small disposable cuvette at room temperature ($20\pm2°C$) and measurements were carried out using the following settings: material refractive index 0.192, absorption 0.1, dispersant refractive index 1.330, viscosity 1.00331 mPa.s.

Without modification, the primary particles of the materials used herein have metal oxide cores and metal hydroxide surfaces and within different disciplines may be referred to as metal oxides or metal hydroxides. The use of the term 'oxo-hydroxy' or 'oxo-hydroxide' is intended to recognise these facts without any reference to proportions of oxo or hydroxy groups. Hydroxy-oxide could equally be used therefore. For the avoidance of doubt, copper hydroxide also includes various chloride-doped polymorphs; in particular, $Cu_2(OH)_3Cl$ is a copper hydroxide derivative in which a chlorine atom was incorporated in the crystalline structure that presents four types of mineral phase: atacamite, botallackite, paratacamite and clinoatacamite. The present inventors believe that the copper oxo-hydroxide nanoparticles compositions of the present invention are altered at the level of the primary particle of the metal oxo-hydroxide with at least some of the ligand L being introduced into the structure of the primary particle, i.e. leading to doping of the primary particle by the ligand molecules. This may be contrasted with the formation of nano-mixtures of metal oxo-hydroxides and an organic molecule in which the structure of the primary particles is not so altered and the organic ligand is only coated or adsorbed on the surface of the particles, as happens when the metal oxo-hydroxide particles are preformed prior to being contacted with the ligand.

The primary particles of the ligand-modified poly oxo-hydroxy metal ion materials described herein may conveniently be produced by precipitation. The use of the term precipitation often refers to the formation of aggregates of materials that do separate from solution by sedimentation or centrifugation. Here, the term "precipitation" is intended to describe the formation of all solid phase material, including aggregates as described above and solid materials that do not aggregate but remain as non-soluble moieties in suspension, whether or not they be particulate or nanoparticulate (colloidal or sub-colloidal). These latter solid materials may also be referred to as aquated particulate solids.

In the present invention, reference may be made to the modified metal oxo-hydroxides having polymeric structures that are not generally crystalline and so have three dimensional polymeric or cross-linked structures that generally form above the critical precipitation pH. As used herein, this should not be taken as indicating that the structures of the materials are polymeric in the strict sense of having a regular repeating monomer unit because, as has been stated, ligand incorporation is, except by co-incidence, non-stoichiometric. The ligand species is introduced into the solid phase structure by substituting for oxo or hydroxy groups leading to a change in solid phase order. In some cases, for example the production of the copper oxo-hydroxide nanoparticle compositions exemplified herein, the ligand species L may be introduced into the solid phase structure by the substitution of oxo or hydroxy groups by ligand molecules in a manner that decreases overall order in the solid phase material. While this still produces solid ligand modified poly oxo-hydroxy metal ion materials that in the gross form have one or more reproducible physico-chemical properties, the materials have a more amorphous nature compared, for example, to the structure of the corresponding unmodified metal oxohydroxide. The presence of a more disordered or amorphous structure can readily be determined by the skilled person using techniques well known in the art. One exemplary technique is Transmission Electron Microscopy (TEM). High resolution Transmission Electron Microscopy allows the crystalline pattern of the material to be visually assessed. It can indicate the primary particle size and structure (such as d-spacing), give some information on the distribution between amorphous and crystalline material. Using this technique, it is apparent that the chemistry described above increases the amorphous phase of our described materials compared to corresponding materials without the incorporated ligand. This may be especially apparent using high angle annular dark field aberration-corrected scanning transmission electron microscopy due to the high contrast achieved while maintaining the resolution, thus allowing the surface as well as the bulk of the primary particles of the material to be visualised.

The combination of these physicochemical properties described above promotes rapid release of copper ions, and as shown in the examples translates into high bactericidal efficacy against a broad range of both gram negative and gram positive bacteria. Importantly, oxo-hydroxides modified with carboxylates, unlike copper salts (such as $CuCl_2$)

or commercial copper nanoparticles, were able to release copper at biocidal levels when incorporated in a delivery matrix such as hydroxyethyl cellulose gel (an example of a topical delivery matrix), showing that ligand functionalisation can be used for the development of topical biocides or to provide a composition that is capable of providing an antibacterial coating for articles.

Examples of properties that can be usefully modulated using the present invention include: dissolution (rate, pH dependence and [Cu] dependence), disaggregation, adsorption and absorption characteristics, reactivity-inertness, melting point, temperature resistance, particle size, magnetism, electrical properties, density, light absorbing/reflecting properties, hardness-softness, colour and encapsulation properties. In this context, a property or characteristic may be reproducible if replicate experiments are reproducible within a standard deviation of preferably $\pm$ 10%, and more preferably $\pm$ 5%, and even more preferably within a limit of $\pm$ 2%. In particular, the present inventors have found that properties of the copper oxo-hydroxide nanoparticles such as lability are retained upon resuspending compositions that have been dried, for example for storage.

The dissolution profile of the ligand modified copper oxo-hydroxide nanoparticles compositions can be represented by different stages of the process, namely disaggregation and dissolution. The term dissolution is used to describe the passage of a substance from solid to soluble phase. More specifically, disaggregation is intended to describe the passage of the materials from a solid aggregated phase to an aquated phase that is the sum of the soluble phase and the aquated particulate phase (i.e. solution plus suspension phases). Therefore, the term dissolution as opposed to disaggregation more specifically represents the passage from any solid phase (aggregated or aquated) to the soluble phase.

*The Ligand (L)*

In the ligand modified copper oxo-hydroxide nanoparticles compositions represented by the formula $(M_x\text{-}L_y(OH)_n)$, L represents one or more ligands or anions, such as initially in its protonated or alkali metal form, that can be incorporated into the solid phase ligand-modified poly oxo-hydroxy metal ion material. In the materials described herein, at least one of the ligands is a carboxylic acid ligand, or an ionised form thereof (i.e., a carboxylate ligand), such as tartarate (or tartaric acid), gluconate (or gluconic acid), adipate (or adipic acid), glutathione and/or an amino acid and/or a sugar acid. Preferably, the ligand is a mono or dicarboxylic acid ligand, and may be represented by the formula $HOCH_2\text{-}R_1\text{-}COOH$ or $HOOC\text{-}R_1\text{-}COOH$ (or an ionised form thereof), where $R_1$ is an optionally substituted $C_{1\text{-}10}$ alkyl, $C_{1\text{-}10}$ alkenyl or $C_{1\text{-}10}$ alkynyl group. In general, the use of ligands in which $R_1$ is a $C_{1\text{-}10}$ alkyl group, and more preferably is a $C_{2\text{-}6}$ alkyl group, is preferred. Preferred optional substituents of the $R_1$ group include one or more hydroxyl groups, for example as present in malic acid. In preferred embodiments, the $R_1$ group is a straight chain alkyl group. A more preferred group of carboxylic acid ligands include tartaric acid (or tartarate), gluconate (or gluconic acid), adipic acid (or adipate), glutaric acid (or glutarate), pimelic acid (or pimelate), succinic acid (or succinate), and malic acid (or malate), and combinations thereof. Whether the carboxylic acid ligand is present as the acid or is partially or completely ionised and present in the form of a carboxylate anion will depend on a range of factors such as the pH at which the material is produced and/or recovered, the use of post-production treatment or formulation steps and how the ligand becomes incorporated into the poly oxo-hydroxy metal ion material. In some embodiments, at least a proportion of the ligand will be present in the carboxylate form as the material are typically recovered at pH>4 and because the interaction between the ligand and the positively charged iron would be greatly enhanced by the presence of the negatively charged carboxylate ion. For the avoidance of doubt, the use of carboxylic acid ligands in accordance with the present invention covers all of these possibilities, i.e. the ligand present as a carboxylic acid, in a non-ionised form, in a partially ionised form (e.g., if the ligand is a dicarboxylic acid) or completely ionised as a carboxylate ion, and mixtures thereof.

Typically, ligands are incorporated in the solid phase poly oxo-hydroxy metal ion materials to aid in the modification of a physico-chemical property of the solid material, e.g. as compared to a poly oxo-hydroxylated metal ion species in which the ligand(s) are absent. In some embodiments of the present invention, the ligand(s) L may also have some buffering capacity. Examples of ligands that may be employed in the present invention include, but are by no means limited to: carboxylic acids such as tartaric acid, gluconic acid, adipic acid, glutaric acid, malic acid, succinic acid, aspartic acid, pimelic acid, citric acid, , lactic acid or benzoic acid; food additives such as maltol, ethyl maltol or vanillin; amino acids such as tryptophan, glutamine, proline, valine, or histidine; and nutrient-based ligands such as folate, ascorbate, pyridoxine or niacin or nicotinamide; sugar acids such as gluconic acid. Typically, two ligands of differing affinities for the metal ion are used in the production of these materials although one, two, three, four or more ligands may be useful in certain applications.

For many applications, ligands need to be biologically compatible under the conditions used and generally have one or more atoms with a lone pair of electrons at the point of reaction. The ligands include anions, weak ligands and strong ligands. Ligands may have some intrinsic buffering capacity during the reaction. Without wishing to be bound by a particular explanation, the inventors believe that the ligands have two modes of interaction:

(a) substitution of oxo or hydroxy groups and, therefore, incorporation with a largely covalent character within the material and (b) non-specific adsorption (ion pair formation). These two modes likely relate to differing metal-ligand affinities (i.e. strong ligands for the former and weak ligands/anions for the latter). There is some evidence in our current work that the two types of ligand are synergistic in modulating dissolution characteristics of the materials and, perhaps, therefore, in determining other characteristics of the material. In this case, two ligand types are used and at least one (type (a)) is demonstrable as showing metal binding within the material. Ligand efficacy, probably especially for type (b) ligands, may be affected by other components of the system, particularly electrolyte.

The ratio of the metal ion(s) to the ligand(s) (L) is also a parameter of the solid phase ligand-modified poly oxo-hydroxy metal iron material that can be varied according to the methods disclosed herein to vary the properties of the materials. Generally, the useful ratios of Cu:L will be between 10:1, 5:1, 4:1, 3:1, 2:1 and 1:1 and 1:2, 1:3, 1:4, 1:5 or 1:10, and preferably ratios of Cu to ligand of 1:1 or lower.

*Producing and processing the copper oxo-hydroxide nanoparticle compositions*

Generally, the copper oxo-hydroxide nanoparticle compositions of the present invention may be produced by a process comprising:

(a) mixing the solution comprising $Cu^{3+}$ and a carboxylic acid ligand, and optionally any further ligands or other components, in a reaction medium at a first pH(A) at which the components are soluble;
(b) changing the pH(A) to a second pH(B) to cause a solid precipitate or a colloid of the copper oxo-hydroxide nanoparticle composition to be formed;
(c) separating, and optionally drying and/or formulating, the copper oxo-hydroxide nanoparticle composition produced in step (b).

Examples of conditions that may be employed include the following using a first pH(A) which is less than 4.0 and the second pH(B) which is between 5.0 and 12.0, and more preferably between 6.0 and 8.0, and carrying out the reaction at room temperature (20-25°C). In general, it is preferred that in step (a), the solution contains 20 to 100mM or 1M $Cu^{2+}$ and 50 to 250mM of a suitable carboxylic acid ligand, and more preferably about 40mM $Cu^{2+}$ and about 100mM of the ligand.

The separation of a candidate material may then be followed by one or more steps in which the material is characterised or tested. By way of example, the testing may be carried out in *vitro* or *in vivo* to determine one or more properties of the material as described above, most notably its dissolution profile, release of soluble copper and/or antibacterial properties. Alternatively or additionally, the process may comprise chemically, e.g. through a titration process, or physically, e.g. through a micronizing process, altering the final particle size of the copper oxo-hydroxide nanoparticle composition and/or subjecting the composition to one or more further processing steps on the way to producing a final composition, e.g. for administration to a subject. Examples of further steps include, but are not limited to: washing, centrifugation, filtration, spray-drying, freeze-drying, vacuum-drying, oven-drying, dialysis, milling, granulating, encapsulating, tableting, mixing, compressing, nanosizing and micronizing.

In some embodiments, additional steps may be carried out between the initial production of the material and any subsequent step in which it is formulated as a medicament. These additional post-production modification steps may include the step of washing the material, to remove impurities or replace an incorporated ligand with the further ligand.

*Hydroxy and oxo groups*

The present invention may employ any way of forming hydroxide ions at concentrations that can provide for hydroxy surface groups and oxo bridging in the formation of these poly oxo-hydroxy materials. Examples include but are not limited to, alkali solutions such as sodium hydroxide, potassium hydroxide sodium phosphate and sodium bicarbonate, that would be added to increase [OH] in an ML mixture, or acid solutions such as mineral acids or organic acids, that would be added to decrease [OH] in an ML mixture.

The conditions used to produce the copper oxo-hydroxide nanoparticle compositions of the present invention may be tailored to control the physico-chemical nature of the precipitate, or otherwise assist in its collection, recovery or formulation with one or more excipients. This may involve purposeful inhibition of agglomeration, or the used drying or grinding steps to subsequently affect the material properties. However, these are general variables to any such system for solid extraction from a solution phase. After separation of the precipitated material, it may optionally be dried before use or further formulation. The dried product may, however, retain some water and be in the form of a hydrated solid phase ligand-modified poly oxo-hydroxy metal ion material. It will be apparent to those skilled in the art that at any of the stages described herein for recovery of the solid phase, excipients may be added that mix with the ligand-modified poly oxo-hydroxy metal ion material but do not modify the primary particle and are used with a view to optimising formulation for the intended function of the material. Examples of these could be, but are not limited to, glycolipids, phospholipids (e.g. phosphatidyl choline), sugars and polysaccharides, sugar alcohols (e.g. glycerol), polymers (e.g. polyethyleneglycol (PEG)) and taurocholic acid.

*Formulations and Uses*

The copper oxo-hydroxide nanoparticle composition of the present invention may be formulated for use as antibacterial agents or antimicrobial agents, for example for the treatment or prevention of bacterial or microbial infections. Accordingly, the compositions of the present invention may comprise, in addition to one or more of the solid phase materials, a pharmaceutically acceptable excipient, carrier, buffer, stabiliser or other materials well known to those skilled in the art. Such materials should be non-toxic and should not significantly interfere with the efficacy of the solid phase materials for the application in question.

The term "antibacterial" as used herein includes the treatment or prevention of infections caused by gram negative and gram positive microorganisms including *Escherichia sp., such as E. coli, Staphylococcus sp., such as S. epidermis, S. aureus* and meticillin-resistant staphylococcus aureus ("MRSA"), *Bacillus sp.,* such as *B. subtilis, Pseudomonas sp., such as P. aeruginosa, Vibrio sp.,* such as *V. fisheri, Streptococcus sp., such as S. pyrogenes* and *S. pneumoniae, Klebsiella sp., Micrococcus sp., such as M. luteus, Clostridium sp.* such as *C. difficile, Acinetobacter sp.* such as *A. baumannii, Mycobacterium sp.,* such as *M. tuberculosis* and *Salmonella sp,* or fungi including *Candida sp., such as C. albicans.* The term "antimicrobial" as used herein is understood to apply to substances including those which inhibit microbial attachment to surfaces, kill microbes and/or inhibit microbial reproduction. The term "microbe" is understood to include all microorganisms, including bacteria as set out above, as well as fungi such as yeast, archaea and protists. The terms "microbial" and "antimicrobial" should be interpreted accordingly.

The use of the copper oxo-hydroxide nanoparticle compositions of the present invention will very depending on whether the compositions are intended for the treatment or prevention of infection in a human or animal subject, or to provide a surface of an article that is resistant to bacterial or microbial colonisation. Example of the latter application include providing coatings for medical equipment or dressings.

In embodiments in which the compositions are intended for the administration to subject, for example in the treatment of wounds or skin infections, the precise nature of the carrier or other component may be related to the manner or route of administration of the composition, typically via a topical route. This may include formulation of the nanoparticle compositions in a solid, semi-solid or gel matrix or in a liquid carrier such as water, petroleum, animal or vegetable oils, mineral oil or synthetic oil. Examples of carriers include physiological saline solution, dextrose or other saccharide solution or glycols such as ethylene glycol, propylene glycol or polyethylene glycol may be included.

The materials and compositions used in accordance with the present invention that are to be given to an individual are preferably administered in a "prophylactically effective amount" or a "therapeutically effective amount" (as the case may be, although prophylaxis may be considered therapy), this being sufficient to show benefit to the individual clinical state. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, Lippincott, Williams & Wilkins. A composition may be administered alone or in combination with other treatments, either simultaneously or sequentially, dependent upon the condition to be treated.

In one example, the copper oxo-hydroxide nanoparticle compositions of the present invention are formulated in a matrix, for example a hydroxyalkyl cellulose matrix, such as hydroxyethylcellulose (HEC) or hydroxymethylcellulose (HMC), or a polyalkylene glycol matrix, such as PEG. Some of these matrices are cellulose derivatives that have been widely used in health care products and cosmetics and have the advantage that they do not require further processing (e.g. heating and drying) of nanoparticles during matrix preparation, thereby having a minimal effect on the antibacterial or antimicrobial properties of the nanoparticle compositions.

In other embodiments, the copper oxo-hydroxide nanoparticle compositions of the present invention may be formulated for topical administration, e.g. in the form of a solid or semi-solid ointment useful in the treatment of wounds, ulcers or the treatment or prevention of bacterial infection. In such applications, polyalkylene glycols are well suited for topical delivery of the materials as they form a cream or an ointment and is available in a range of different molecular weights, allowing the tailoring of viscosity and other physical parameters that may be desirable in the final ointment. The application of the present invention to topical products has therapeutic use for wound healing and as in anti-infective compositions.

In all aspects of the present invention in which the compositions are formulated for administration to a subject, it is preferred that the pH of the composition or a formulation containing it is raised to a physiological pH, preferably to a pH between 5.0 and 9.0, and more preferably to a pH of between 6.0 and 8.5. The examples show that the compositions of the present invention are capable of making free copper bioavailable under these conditions. Conveniently, this may be done by adding a base, such as sodium hydroxide or sodium carbonate. The aim of this is so that administration to a subject will not result in unintended clinical outcomes, such as pain or inflammation.

An effective amount of copper oxo-hydroxide nanoparticle compositions herein may be formulated for topical application, e.g. to the skin, teeth, nails or hair. These compositions can be in the form of creams, lotions, gels, suspensions, dispersions, microemulsions, nanodispersions, microspheres, hydro gels, emulsions (oil-in-water and water-in-oil, as well as multiple emulsions) and multilaminar gels and the like (see, for example, The Chemistry and Manufacture of Cosmetics, Schlossman et al., 1998), and may be formulated as aqueous or silicone compositions or may be formulated as emulsions of one or more oil phases in an aqueous continuous phase (or an aqueous phase in an oil phase). The type of carrier utilized in the present invention depends on the properties of the topical composition. The carrier can be solid, semi-solid or liquid. Suitable carriers are liquid or semi-solid, such as creams, lotions, gels, sticks, ointments, pastes, sprays and mousses. Specifically, the carrier is in the form of a cream, an ointment, a lotion or a gel, more specifically one which has a sufficient thickness or yield point to prevent the particles from sedimenting. The carrier can itself be inert or it can possess benefits of its own. The carrier should also be physically and chemically compatible with the antibacterial composition or other ingredients formulated in the carrier. Examples of carriers include water, hydroxyethyl cellulose, propylene glycol, butylene glycol and polyethylene glycol, or a combination thereof.

In addition to the therapeutic use of the ligand modified copper oxo-hydroxide nanoparticle composition, they may also be applied as antimicrobial or antibacterial coatings to articles, for example coatings on substrates which comprise woven fabric, non-woven fabric, plastic, glass and/or metal. The antimicrobial nature of the coatings makes them particularly suitable to be applied to substrates for use in medical or personal care applications. In particular, the coatings are particularly useful on substrates which are in contact with the body, for example with skin or mucous membrane, in normal use, for example dressings, bandages and plasters.

For example, microbial growth is a particular problem when skin or mucous membrane is covered, for example by a wound dressing, nappy or underwear. As soon as skin or mucous membrane becomes covered, the environmental conditions for microbial growth improve. Microbes present on the covered skin or mucous membrane can multiply at enhanced rates, particularly when the environment is moist and/or not exposed to air. Secretions from these microbes include acid or alkali excretions which can alter the pH of the skin, toxin secretion and enzyme secretion, including protease secretion. These secretions and excretions can cause skin and mucous membrane irritation, and in the more severe cases skin or mucous membrane breakdown, such as dermatitis.

Particular conditions which can occur following to the covering of skin or mucous membrane include thrush. Thrush is a fungal infection, by the *Candida* genus of yeast, particularly *Candida albicans.* Symptoms include itching, burning and soreness, and inflammation of the infected area. The wearing of sanitary towels, incontinence pads, nappies and/or tight underwear can produce conditions favourable to *Candida* growth, which can lead to thrush. The coatings used in the present invention may be effective against fungi such as yeast, and accordingly it will be understood that providing the coatings on the above mentioned items may enable the treatment and/or prophylaxis of thrush.

Similarly, contact dermatitis (commonly known as nappy rash) may be caused by the wearing of incontinence pads or nappies. Damp or wet skin loses its structure, high pH can promote bacterial growth and the bacteria can secrete enzymes which break down the skin tissue. This environment can also promote or exacerbate pressure ulcers (commonly known as bed sores), which are particularly problematic when they become infected. The coatings have been found to be effective against bacteria, and accordingly it will be understood that providing the coatings on tampons, sanitary towels, incontinence pads or nappies may enable the treatment and/or prophylaxis of contact dermatitis and/or pressure ulcers.

For similar reasons, contact dermatitis and yeast infections can occur under medical dressings, for example dressings for wounds and burns. An additional consideration with medical dressings is the need to prevent bacterial infection of the wound or burn. When skin is burnt, a large amount of tissue may be damaged which can reduce or destroy the natural barrier properties of skin, and wounds which break the skin also affect the barrier properties of skin. This can lead to opportunistic infection that can delay healing, and to septic shock. Additionally, microbial infection, particularly bacterial infection, can be a problem after surgery. The use of medical or surgical devices, for example implantable medical devices, which are coated with the present antimicrobial coatings may help to prevent or treat post-surgical infection. Accordingly, it will be understood that providing the coatings on dressings for wounds and/or burns may enable the treatment and/or prophylaxis of contact dermatitis and/or microbial infection.

The copper oxo-hydroxide nanoparticle compositions of the present invention, then, can be used in the manufacture of a medicament for the treatment and/or prophylaxis of microbial infection, and/or of skin or mucous membrane disorders such as inflammation and dermatitis. In particular, the antibacterial or antimicrobial coatings may be useful for the treatment and/or prophylaxis of infection of a wound, infection of a burn, infection of a pressure ulcer, post-surgical infection, thrush, contact dermatitis and pressure ulcers. The microbial infection may be by any microbe, in particular bacteria and/or yeast such as *Staphylococcus sp.,* such as *S. aureus, Pseudomonas sp.,* such as *P. aeruginosa, Micrococcus sp.,* such as *M. luteus, Saccharomyces sp.,* such as *S. cerevisiae, Candida sp.,* such as *C. albicans, Staphylococcus sp.,* such as *S. epidermis, Streptococcus sp.,* such as *S. pyrogenes, Klebsiella sp.* and *Escherichia sp.,*

such as *E. coli, Chlamydia sp.* The compositions may further be active against viruses or parasites.

The medicament may be a substrate coated by the coating methods of the present invention. For example, then, the medicament may be a coated substrate such as a coated medical device, for example an implantable medical device. Examples include a surgical seed, catheter (such as a urinary catheter, a vascular access catheter, an epidural catheter), a vascular access port, an intravascular sensor, a tracheotomy tube, a percutaneous endoscopic gastrostomy tube, an endotracheal tube, an implantable prosthetic device, such as a stent and related short-indwelling or biocontacting devices. The medicament may be a coated substrate such as a coated nappy, sanitary towel, tampon, incontinence pad, dressing such as a wound or burn dressing, bandages or underwear. Many of these substrates (particularly nappies, sanitary towels, incontinence pads and dressings such as wound or burn dressings) comprise a non-woven fabric component, which may be in contact with skin or mucous membrane in normal use. The present inventors have demonstrated that the coatings and coating methods of the present invention are particularly suited to non-woven fabric substrates.

As used herein, the term "non-woven fabric" includes fabrics or textiles formed from a web of fibres. In non-woven fabric, the fibres are not woven or knitted. Non-wovens are typically manufactured by putting small fibers together in the form of a sheet or web, and then binding them mechanically. Example non-woven fabrics include polypropylene non-wovens.

It will be understood that the manufacturing process of the medicament may include providing an antimicrobial coating on a substrate. Accordingly, the manufacture of the medicament may comprise any of the steps of the methods described herein for providing antimicrobial coatings.

The present invention also provides substrates coated by the present methods. The coated substrates may be for use in a method of medical treatment, and include the coated substrates mentioned above as possible medicaments. It will be understood that the present invention also provides a method of medical treatment for the treatment and/or prophylaxis of microbial infection and/or of disorders of the skin or mucous membrane, and the use of the present coated substrates in such methods. The coating methods of the present invention are applicable to coating the substrates mentioned herein, as medicaments or otherwise.

As well as the applications described above, the antimicrobial coatings may also be provided on other equipment for use in medical applications, for example in hospitals. There is significant interest in controlling infection in hospitals, in particular bacterial infection such as MRSA and *Clostridium difficile.* As discussed above, microbial colonisation of surfaces is a particular problem. However, the present coatings have been found to be effective against many species of microbe, and so it will be understood that providing the present antimicrobial coatings on the surface of hospital equipment may be beneficial. Accordingly, substrates which may be coated according to the present invention include medical equipment and devices which contact the body or body fluids in normal use. For example, suitable substrates include tubes, fluid bags, catheters, syringes and surgical equipment such as scalpels and forceps etc. Additionally, other equipment, for example equipment used in hospitals (e.g. healthcare equipment) may be coated according to the present invention, for example gowns (e.g. surgical gowns), surgical masks, protective gloves (e.g. surgical and examination gloves), curtains, uniforms and bedding such as pillow cases, waterproof mattress covers (for example in babies cots and intensive care beds) and sheets.

Alternative healthcare equipment includes surgical draperies, surgical socks, furniture such as tables including bedside tables, beds, and seating surfaces, and other equipment including storage containers, filters, and service trays.

Additionally, the coatings are useful in coating equipment which it is desirable to keep free of microbes, for example equipment which is used in processing of food, for example kitchen equipment and surfaces, and factory equipment used in the manufacture or processing of food. For example, substrates which can be coated according to the present invention include containers (such as food storage containers), conveyors, blades, mixers, rollers and kitchen utensils (such as cutting and serving implements). Additional substrates include food preparation surfaces, flexible and rigid packaging and door handles.

Additionally, protective clothing worn by workers, for example overalls, gloves, masks and hats could be coated. Other clothing which may be coated includes undergarments, socks, athletic apparel, surgical apparel, healthcare apparel, shoes and boots.

Other substrates suitable for coating include filters, for example medical filters (including respirator filtration media and fluid filtration media), and other filters including HVAC filtration media, water filtration media and fluid filtration media.

Further suitable substrates include currency, debit/credit cards, industrial waste and water handling equipment, petrochemical and crude oil production, distribution and storage equipment and infrastructure. Additional suitable substrates include personal protective equipment and military apparatus such as face masks, respirators, decontamination suits and gloves.

**Experimental Examples**

All experiments were carried out using ultra high pure (UHP) water (distilled deionised water; 18.2 $\Omega$M /cm), and at room temperature (20 $\pm$ 2°C), unless otherwise stated.

### *1.1 Synthesis and preparation of copper materials*

#### *1.1.1 Copper chloride stock solution*

$CuCl_2.2H_2O$ was dissolved in UHP water to produce a concentrated stock at 40mM (2542ppm Cu), which was used in subsequent assays.

#### *1.1.2 CuO nanoparticles*

Commercial CuO nanoparticles were obtained from Sigma-Aldrich (544868) and used as received and used for comparison with the antibacterial compositions of the present invention. Stock suspensions were prepared by dispersing the nanopowders in UHP water at 20mM (1270ppm Cu).

#### *1.1.3 Silicate-stabilised copper hydroxide nanoparticles (CuSi NPs)*

CuSi nanoparticles were prepared for comparative purposes by mixing a 400mM sodium silicate solution at ca. pH 12, with a copper chloride solution (40mM Cu), in a volume ratio of 1:1. The resulting suspension, containing 20mM Cu and 200mM Si, was pH adjusted to 12 $\pm$ 0.2 with 5M NaOH, and was kept under stirring for 24 hours. After this period a light blue clear solution had been formed.

#### *1.1.4 Cu-EDTA complexes*

Cu-EDTA complexes were freshly prepared by dissolving $CuCl_2.2H_2O$ and disodium ethylenediaminetetraacetate (EDTA) di-hydrate in UHP water. The pH was adjusted to 7.5 $\pm$ 0.2 with 1M NaOH. Various Cu:EDTA ratios were achieved by maintaining concentration copper at 20mM (ca. 1270ppm), whilst changing that of EDTA - 20, 100 and 200mM - thus achieving Cu-EDTA ratios of 1:1, 1:5 and 1:10, respectively. Copper solubility was confirmed by ICP-OES using elemental phase distribution (see 2.4.1.).

#### *1.1.5 Tartrate Adipate modified copper oxo-hydroxide nanoparticles (CuTartAd NPs)*

An acidic solution comprising 40mM copper chloride, 20mM adipic acid and 20mM tartaric acid was prepared. The pH of this, initially acidic, solution was raised through drop-wise addition of 5M NaOH up to pH 8.2 $\pm$ 0.2. The final suspension contained ca. 40mM (2500ppm) Cu.

Nanoparticles synthesised as per Example 1.1.5 were characterised for copper phase distribution. During the synthetic process, soluble copper converted to particulate copper oxo-hydroxide as pH increased. Above pH 5, the particulate phase was mostly composed of nanoparticles (fraction greater than 80% of total particulate).

### *1.2 Nanoparticle characterisation*

#### *1.2.1 Hydrodynamic particle size distribution*

Hydrodynamic particle size distribution of nanoparticles was determined by Dynamic Light Scattering (DLS) on a Zetasizer NanoZS (Malvern Instruments). In a typical experiment, 0.5 to 1 ml of nanoparticulate suspension (as prepared in 2.1.) was transferred into a small disposable cuvette at room temperature (20$\pm$2°C) and 3 measurements were carried out using the following settings:

| | |
|---|---|
| Material Refractive Index | 0.192 |
| Absorption | 0.1 |
| Dispersant Refractive Index | 1.330 |
| Viscosity | 1.00331 mPa.s |

*1.2.2 Zeta Potential*

Zeta potential was analysed on a Zetasizer NanoZS (Malvern Instruments), by Laser Doppler Micro-electrophoresis, using a dielectric constant of 78.5 and a viscosity of 0.89cP. Nanoparticle suspensions at ca. 1270ppm Cu were transferred into clear disposable zeta cells to perform the measurement.

*1.2.3 Transmission Electron Microscopy (TEM)*

A suspension of CuTartAd NPs containing 2500ppm Cu was analysed by TEM. TEM grids were prepared by dispersing the nanoparticulate suspension in methanol and drop-casted on holey carbon film TEM grids (Agar Scientific). Images were obtained on a CM200 (S)TEM fitted with an Oxford Instruments X-Max 80 mm2 SD detector and AZTEC analysis software.

*1.2.4 X-Ray Diffraction (XRD) analysis*

CuTartAd NPs were dried at 45°C for 24 hours and manually milled prior to conventional X-Ray Diffraction (XRD) analysis.

***1.3 Bacterial work***

*1.3.1 Heavy Metal MOPS (HMM) medium, pH 7.2 $\pm$ 0.2.*

HMM is a copper-free defined medium developed for testing heavy metals and here was supplemented with glucose and cas-amino acids (acid hydrolysate of casein) to provide all basic nutrients required for bacterial growth. HMM was prepared from concentrated stock solutions of each reagent, and pH adjusted to 7.2 $\pm$ 0.2 (Table 1). Freshly prepared medium was immediately autoclaved at 121°C for 15 minutes, let cool down and stored at 4 $\pm$ 2°C. Autoclaved medium was used within a month from preparation.

**Table 1.** Composition of HMM medium.

| Reagent | Concentration in HMM medium |
| --- | --- |
| 3-(N-morpholino)propanesulfonic acid (MOPS) | 40mM |
| KCl | 50mM |
| $NH_4Cl$ | 10mM |
| $MgSO_4$ | 0.5mM |
| $FeCl_3.6H_2O$ | 1$\mu$M |
| Glycerol-2-Phosphate | 1mM |
| Glucose | 0.4% (w/v) |
| Casein acid hydrolysate | 0.1% (w/v) |

*1.3.2 Bacterial growth inhibition assay*

Antimicrobial activity was assessed through determination of bacterial growth inhibition in the presence of copper compounds. A turbidimetric assay was used to follow bacterial concentration over time as this is proportional to optical density (OD at 595nm) in liquid medium, allowing an easy screening of bacterial growth over time. *Escherichia coli* NCTC11100 and *Staphylococcus aureus* RN4220 were the tested microorganisms in this assay. Stock bacterial colonies were kept in cultivated in agar plates and on the day before the experiment, one colony was transferred into 10ml of HMM liquid medium and grown overnight at 30°C under constant shaking (80rpm) in an incubator. On the day of the assay, the OD of the bacterial suspension was measured at 595nm on a plate reader (Multiskan RC 351, Labsystems) and diluted in HMM to achieve an OD between 0.05 and 0.10 (CFU), ensuring that the initial concentration of bacteria was kept constant throughout the assays. Copper stock solutions (refer to section 2.1) were sequentially diluted in HMM to achieve typical concentrations between 0.8 and 100ppm Cu in a volume of 0.1ml. Next, 0.1ml of bacterial culture was added and incubated with copper at 30°C under constant agitation (80 rpm). Final copper concentrations in the assay ranged between 0.4 and 50ppm, and OD was measured every hour for a typical period of 7-8 hours to follow bacterial growth. OD background, i.e. OD absorbance not caused by bacteria, was determined to remove readout interference from copper and broth. Growth inhibition was calculated as follows:

$$\text{Growth Inhibition } \% = \left( \frac{OD\ Control - OD\ Copper}{OD\ Control} \right) \times 100$$

OD control: OD of bacteria incubated in HMM in the absence of copper, after subtraction of OD of medium (no bacteria).
OD copper: OD of bacteria incubated in HMM in the presence of copper, after subtraction of OD of medium plus a matching concentration of copper (no bacteria).

### 1.3.3 Copper-bacteria association

The association of copper with *E. coli* NCTC11100 cultures was studied by initially growing the bacterial cultures overnight in HMM at pH 7.2±0.2 (as described in 2.3.2), to reach the stationary phase, such that bacterial concentrations remained constant throughout the assay. This concentration was determined to be 9x108 CFU/ml by agar plate counting. Next, copper chloride stock solution was diluted in the bacterial cultures to 3 and 12.5 ppm Cu, and incubated at 30°C. Samples were collected at 0, 2, 4 and 8 hours. At each time point, one aliquot of each sample was used to determine total copper concentration, and a second one was centrifuged for 5 min at 16000g on a benchtop Biofuge Pico (Heraeus), to sediment bacteria and associated copper. Free copper in the supernatant was analysed by inductively coupled plasma-optical emission spectroscopy (as in 2.4.1.). Lastly, copper associated to bacteria was determined as below:

$$[Cu]\ associated\ to\ bacteria = Total\ [Cu] - Supernatant\ [Cu]$$

### 1.4 Chemical assays

#### 1.4.1 Elemental copper analysis

Inductively coupled plasma-optical emission spectroscopy (ICP-OES) was used to determine elemental copper concentration. All samples were diluted in 5% HNO3 (v/v) at least 24 hours prior to analysis to dissolve copper materials. Calibration standards were matrix-matched in 5% $HNO_3$, ranging from 0.1 to 100 ppm. The line used for copper detection was 324.754 nm.

#### 1.4.2 Determination of copper phased distribution: soluble, nanoparticulate and microparticulate fractions

Phase distribution was determined by separating soluble (<1.4nm), nanoparticulate (<100nm) and submicron/microparticulate (>100nm) copper. Three samples were collected, 1) Total, analysed neatly for copper concentration; 2) supernatant, centrifuged for 5 minutes at 16000g, followed by supernatant analysis; and 3) soluble, filtered through a 3KDa filter. Phase distribution was then calculated as:

$$Copper\ Microparticulate\ \% = \frac{Cu_{Total} - Cu_{Supernatant}}{Cu_{Total}} \times 100$$

$$Copper\ Soluble\ \% = \frac{Cu_{Soluble}}{Cu_{Total}} \times 100$$

$$Copper\ Nanoparticulate\ \% = Cu_{Soluble}(\%) - Cu_{Microparticulate}(\%)$$

#### Copper-based nanoparticles dissolution

Dissolution of copper nanoparticles was studied in bacterial growth medium (HMM). Copper materials were diluted from stocks (see section 2.1) to 12.5, 25 and 50ppm copper, and aliquots were collected at 0, 2, 4 and 8 hours. Fraction of soluble copper was determined by analysis of phase distribution as described in described in section 2.4.2.

*1.4.4 Copper dispersibility*

Stock copper materials were diluted in 50mM 3-(N-morpholino)propanesulfonic acid (MOPS) buffer at pH 7.4 $\pm$ 0.2. Final copper concentrations varied between 10 and 500ppm. Microparticulate copper was removed by centrifugation at 16000g for 5 minutes. Total copper and supernatant (i.e. disperse fraction) were analysed by Inductively Coupled Plasma-Optical Emission Spectroscopy (ICP-OES) as described in section 2.4.1.

*1.4.5 Copper release from a gel matrix - Gel Release assay*

$CuCl_2$ and CuTartAd nanoparticles, at pH between 7 and 8, were incorporated into a hydroxyethylcellulose (HEC) matrix by diluting the original stocks containing ca. 2500ppm, down to 250ppm in UHP water, and next dissolving hydroxyethylcellulose (HEC) to achieve a concentration of 2% (w/v). The mixture was kept under moderate stirring in a rotary shaker until a homogeneous gel was formed. 10g of the gel was poured into a 50ml falcon tube and let settle down overnight. 10ml of a 50mM NaHCO3 solution at pH 7 $\pm$ 0.2 were carefully transferred on the surface of the gel. Samples were collected over 24 hours and copper concentration in the overhead solution was determined by ICP-OES (see section 2.4.1.).

### 2. Copper-based nanoparticles as delivery agent for biocidal copper ions

A turbidimetric assay was developed to test antimicrobial activities of nanoparticulate materials in which *E. coli* concentration was followed through optical density measurements in a liquid media that enabled *in situ* characterisation of copper phase distribution. Bacteria were incubated in the presence of a broad range of copper concentrations, and copper chloride was used as a reference biocidal material to provide soluble copper, see Figure 1. The next stage of this work required the selection of appropriate nanoparticulate materials. Initially, commercial CuO nanoparticles with indicated sizes of ca. 50nm were studied; however, when dispersed in water these nanoparticles formed large micron-sized agglomerates (Figure 2A). Their unexpected lack of dispersibility was explained by weak surface repulsion as evidenced by a zeta potential peak of only -7.1$\pm$0.5mV.

Consequently, alternative copper-based nanoparticles were developed to act as genuine nanoparticulate agents, i.e. stable colloids at concentrations suitable for delivery in the assay. Silicate was chosen as stabiliser given its lack of antimicrobial activity, such that particle toxicity would be driven by copper alone. The resulting reference silicate-stabilised copper hydroxide nanoparticles (CuSi NPs) were thus synthesised through co-precipitation, in which a copper chloride solution was mixed with alkaline sodium silicate solution at ca. pH 12. Initially, $Cu^{2+}$ ions precipitated as $Cu(OH)_2$, forming micron-sized agglomerates, but these dispersed over time to form small nanoparticles with a hydrodynamic diameter peak of 8.5$\pm$0.3nm (Figure 2D). This process of dispersion was anticipated to be driven by the adsorption of negatively-charged silicate ions to copper hydroxide agglomerates. Additionally, high ratios of silicate-to-copper (10:1) ensured efficient negative charge repulsion, as confirmed by the Zeta Potential measurement, which showed that the nanoparticles were sufficiently negatively charged (-31$\pm$9mV) to resist agglomeration (Figure 2B).

Next, the antimicrobial activities of these two distinct types of nanoparticle, CuO and CuSi, were compared to a soluble copper control (CuCl2) across a range of copper concentrations (0.8-50ppm Cu) against *E. coli*. Nanoparticles were found to be less efficacious than soluble copper, which is particularly well illustrated at an exposure level of 50ppm Cu (Figure 3). Here, growth inhibition upon exposure to CuSiNPs was more pronounced at the latter time points implying a gradual and delayed effect of CuSi NPs on bacteria. Surprisingly, despite the observed agglomeration, CuO nanoparticles showed equivalent growth inhibition to CuSi NPs at 6 hours. However, the increase in inhibition over time was not as pronounced as for CuSi NPs. Similar biocidal kinetics of nanoparticles, i.e. both showed gradual and delayed growth inhibition, may imply a common nanoparticulate mode of action distinct from that of soluble copper, e.g. adhesion to bacterial membrane. However, such a mechanism seems unlikely given the disparities in physicochemistry between the nanoparticles (e.g. size, charge and composition). For instance, nanoparticles with different surface charge would be expected to show different affinities for the bacterial membrane, and consequently different antimicrobial activities, which were not observed here. More plausibly, a dissolution mediated mechanism based upon the release of copper ions from nanoparticles would explain delayed toxicity relative to copper chloride, and is supported by respective dissolution profiles of both materials. Release of copper ions accorded to growth inhibition, in which faster dissolution of CuO NPs resulted in greater antimicrobial efficacy at earlier time points (2-4 hours). After 4 hours, the percentage of soluble copper released from either of the nanoparticles was equivalent (ca. 40% of total copper) which translated into similar bacterial growth inhibition (ca. 80%). Similarity in dissolution profiles of the two types of nanoparticles was surprising, since smaller CuSi NPs were expected to dissolve faster, due to their higher surface area-to-volume ratio, but the presence of insoluble silicates in the structure of nanoparticles may have retarded dissolution. Critically, the association between dissolution and bacterial growth inhibition implied that biocidal activity was driven by soluble copper for both types of nanoparticles.

To further clarify the 'dissolution' theory, *E. coli* growth inhibition was compared for soluble (<1.1nm) as well as nanoparticulate (1-100nm) copper fraction in the bacterial culture medium. A dose response was observed for CuO materials, in which increasing levels of copper (12.5, 25 and 50ppm) led to an increase in growth inhibition (Figure 4). However, agglomeration resulted in very low concentrations of nanoparticulate copper (<3ppm), and thus the increase in growth inhibition could not be attributed to this fraction, but rather to the increase in soluble copper. CuSi NPs behaved in a distinct manner to commercial CuO NPs: here, greater quantities of material resulted in increased nanoparticulate copper concentrations (3, 10 and 38ppm) but relatively unchanged levels of soluble copper (10-15ppm). However, such increase in nanoparticulate copper did not result in additional biocidal action and, instead, growth inhibition accorded with relatively static levels of soluble copper.

The dependence of biocidal activity upon soluble copper, suggests that optimal antibacterial efficacy would be achieved by copper salts, which readily delivered soluble copper ions in the medium used for the growth inhibition assay (Figure 4). However, use of copper as an antimicrobial agent in clinical applications, such as wound healing (including treatment of cuts and abrasions), requires formulations at concentrations much greater than those tested in the antimicrobial assay (<50ppm) to enable the delivery of quantities of copper that are effective at killing bacteria. In addition, these formulations should be delivered at physiological pHs (pH 6-8) to avoid further detrimental effects of extreme pHs on a skin that is already vulnerable by the wound. Therefore, appropriateness of copper salts in formulations for wound healing was investigated by quantifying their dispersibility, i.e. non-precipitated, in a MOPS buffer at pH 7.4±0.2, as an indication of bioavailability. Most copper precipitated as large centrifugible agglomerates, reducing the dispersible fraction to a maximum of ca. 10ppm of Cu, despite addition of copper at concentrations as high as 500ppm. Therefore, copper salts have limited used in formulations as they are not stable towards precipitation which limits the bioavailable copper.

The undesirable precipitation of copper salts at physiological pH occurred through the formation of large (centrifugible) copper hydroxide agglomerates that can be prevented through the use of complexing agents (e.g. EDTA). For instance, in the growth inhibition assay, copper was maintained soluble in the bacterial medium by complexing agents present in the medium, possibly amino acids. The viability of this strategy was employed at various Cu:EDTA ratios, and the resulting solutions were tested in the bacterial assay (Figure 6A). Despite keeping copper in solution, Cu-EDTA complexes showed modest growth inhibition (<40%; Figure 6B). Interestingly, EDTA alone had antibacterial effect, which arguably could be responsible for most the effect of the complexes. These observations showed that whilst the release of copper ions is essential for antibacterial purposes, the form of soluble copper is also important, and when in the presence of strong complexing agents, such as EDTA, availability of free copper ions is reduced since such strong chelates 'compete' with bacteria for copper, resulting in reduced toxicity.

Despite the delayed growth inhibition observed for copper-based nanoparticles in comparison to copper salts (Figure 3), nanoparticles showed a greater antimicrobial activity than copper complexes, implying a greater capacity to deliver free copper ions. Therefore, their appropriateness for clinical formulations was also tested in the same conditions as described for copper chloride.

In summary, this initial body of work demonstrated that copper-based nanoparticles had little or no direct effect on bacteria, and their biocidal activity was triggered via the release of copper ions, the main biocidal form of copper. Unlike copper salts and copper complexes, which are not appropriate for the release of free copper ions, nanoparticles have great potential as a delivery systems for such species and can remain disperse at suitable concentrations for formulation in clinical applications. However, the CuO and CuSi nanoparticles tested in this work are not optimal as the rate of copper released was found to be low.

### 3. Novel effective copper-based nanoparticles for the delivery of copper ions

#### 3.1 Introduction

As maximum antimicrobial efficacy of copper-based nanoparticles can be achieved through rapid release of copper ions, we attempted to produce such labile materials by modifying their mineral structure via synthetic methodologies that promote the formation of unstable mineral phases.

#### 3.2 Ligand modified copper oxo-hydroxide nanoparticles

Copper oxo-hydroxide minerals were prepared through pH-driven precipitation of a copper chloride solution by drop-wise addition of sodium hydroxide, which forced the conversion of copper ions to copper oxo-hydroxides. This was carried out in the presence of carboxylate ligands, namely tartaric acid and adipic acid, which controlled mineral growth at the nanoscale as a result of ligand incorporation and surface capping of the mineral growth front, to produce small and stable nanoparticles, with core structures of 2 to 5nm (Figure 7). Tartaric acid played a key role in stabilising the nanoparticles in solution via electrostatic repulsion, presumably through its negative carboxylate groups - deprotonated above pH 4.4, its second pKa. Zeta potential showed that nanoparticles were sufficiently negatively-charged (peak at -39mV) to prevent particle aggregation due to strong particle repulsion (|Zeta Potential|>30mV), and therefore the formation of a very stable suspension. XRD analysis indicated an amorphous mineral phase, which is likely due to the incorporation of tartaric acid in the mineral structure. In contrast, the present inventors believe that adipic acid, a weak ligand with low affinity for copper, was mainly used for its buffering capacity to control the pH during the synthesis. The CuTartAd NPs also showed an amorphous mineral phase, likely due to surface disruption of the mineral lattice by tartaric acid. Amorphousness may impact on lability, since materials with amorphous minerals phase are more labile than crystalline ones.

Following synthesis of CuTartAd nanoparticles, their dissolution profile was determined in bacterial growth medium upon dilution to 12.5, 25 and 50 ppm Cu, concentrations normally used in the antimicrobial assays. Nanoparticles dissolved immediately after dilution in the medium (Figure 8), and remained in solution for at least 8 hours, the period studied in this assay. As previously observed for CuSi NPs, CuTartAd NPs were stable in dispersion at high copper concentrations (Figure 10B), but unlike CuSi NPs, were extremely labile, demonstrating rapid release of copper in bacterial growth medium.

Having confirmed lability, antimicrobial efficacy testing of CuTartAd NPs ensued. Two standard bacterial models were used to measure activity against both *E. coli* and *S. aureus,* a gram-negative and a gram-positive bacterium, respectively. CuTartAd NPs were found to be efficacious against both strains, inhibiting *S. aureus* growth by more than 80%, whilst fully inhibiting *E. coli* growth at incubations of 50ppm Cu (Figure 9A). This represented an improvement relative to CuSi NPs, which failed to fully inhibit E. coli growth at the same concentration (Figure 3). These results reinforced the significance of soluble copper ions for antimicrobial effect; both nanoparticles, CuSi NPs and CuTartAd NPs, exhibited similar physicochemical properties (e.g. small size and negative charge), but different dissolution rates and corresponding differences in antibacterial activity. Moreover, CuTartAd NPs showed equal efficacy to soluble copper, demonstrating their suitability for delivery of biocidal copper. The ligand modified copper oxo-hydroxide nanoparticles of the present invention have a bactericidal effect against a broad range of microorganisms, including pathogenic models *of P. aeruginosa* and *S. aureus* (Table 3).

**Table 3.** Minimum bactericidal concentration (MBC) obtained from incubation $CuCl_2$ or CuTartAd with several bacterial models, including conventional lab strains (*E. coli* MC1061 and *B. subtilis* BR151), pathogenic models (*S. aureus* RN4220 and *P .aeruginosa*) and ISO standards for toxicity tests (*V. fischeri*). Each bacterial species was incubated with Cu, both $CuCl_2$ and CuTartAd nanoparticles, in liquid medium from 4 to 96 hours. Next, bacterial cultures were transferred to agar plates and MBC values were determined through visual inspection of colonies formed (n = 1).

| $CuCl_2$ | MBC (ppm Cu) | | | | |
|---|---|---|---|---|---|
| Time (h) | *E. coli* | *B. subtilis* | *S. aureus* | *P. aeruginosa* | *V. fischeri* |
| 4 | 100 | >100 | 100 | 100 | 100 |
| 24 | 50 | >100 | 50 | 100 | 100 |
| 48 | 10 | >100 | 50 | 100 | >100 |
| 72 | 50 | >100 | 50 | 100 | >100 |
| 96 | 50 | >100 | 50 | 100 | >100 |
| CuTartAd | MBC (ppm Cu) | | | | |
| Time (h) | *E. coli* | *B. subtilis* | *S. aureus* | *P. aeruginosa* | *V. fischeri* |
| 4 | 100 | >100 | 100 | 100 | 100 |
| 24 | 100 | >100 | 50 | 100 | 100 |
| 48 | 10 | >100 | 50 | 100 | >100 |
| 72 | 50 | >100 | 50 | 100 | >100 |
| 96 | 10 | >100 | 50 | 100 | >100 |

Broad-spectrum antimicrobial efficacy across gram negative and gram positive species is advantageous for numerous clinical applications, in particular to combat wound infections - due to the variety and number of pathogens wounded skin is exposed to, and the deleterious impacts of infection upon healing. Thus, having demonstrated the biocidal efficacy of CuTartAd NPs, their appropriateness for topical delivery was tested. Typical formulations for wound healing comprise dressings or creams in which actives are impregnated and then released upon exposure to moisture. Here, as a proof of principle, nanoparticles were incorporated in a hydroxyethylcellulose (HEC) matrix. HEC is a cellulose derivative that has been widely used in health care products and cosmetics, and unlike dressings or other matrices (e.g. polyethylene glycol), HEC does not require any further processing (e.g. heating and drying) of nanoparticles during matrix preparation, with minimal alterations to their physicochemical properties. Thus, incorporation of CuTartAd NPs was achieved simply by diluting colloids to the desired concentration and dissolving HEC into the suspension which resulted in the formation of a homogeneous gel that embedded the nanoparticles.

Importantly, copper was released from this gel: over 24 hours, HEC matrices impregnated with CuTartAd NPs at 250ppm were found to release $64 \pm 8$ ppm - a concentration more than sufficient to inhibit bacterial growth. In contrast, gels formulated with 250ppm Cu chloride failed to release more than 10ppm Cu over the same time period. This confirmed the inappropriateness of copper salts as delivery agents at physiological pHs. As such, CuTartAd nanoparticles were here shown to have suitable properties for the combat of wound infections, as they released biocidal copper ions readily, which resulted in high antimicrobial activity and were appropriate for topical delivery.

## 4. Synthetic Examples

### 4.1 CuOH 40 Tart20 Ad20 nanoparticles prepared from $CuSO_4$

Nanoparticles were synthesised as per Example 1.1.5, but $CuSO_4$ was used instead of $CuCl_2$. Nanoparticles synthesised from $CuSO_4$ as per this example were characterised for copper phase distribution. During the synthetic process soluble copper converted to particulate copper oxo-hydroxide as pH increased. By pH 7, the particulate phase was mostly composed of nanoparticles (approximately 80% of total copper). Hydrodynamic particle size was determined by Dynamic Light Scattering during the synthetic process of tartrate-adipate modified copper oxo-hydroxide nanoparticles synthesised as per Example 1.1.5. In addition to increased dispersibility, pH increase resulted in reduced particle size. For instance nanoparticles recovered at pH 6.5 exhibit larger particle sizes ($73 \pm 10$ nm) than particles recovered at higher pHs (e.g. $4.6 \pm 0.5$nm at pH 8). When recovered at pH 8, these had hydrodynamic diameters between 1.5 and 20nm, with mean diameters between 3 and 5nm.

### 4.2 CuOH 40 Tart20 Ad20 nanoparticles prepared from $CuNO_3$

Nanoparticles were synthesised as per Example 1.1.5, but $CuNO_3$ was used instead of $CuCl_2$. Nanoparticles synthesised from $CuNO_3$ as per this example were characterised by Dynamic Light Scattering. When recovered at pH 8, these had hydrodynamic diameters between 2 and 10nm, with mean diameters between 3 and 5nm.

### 4.3 CuOH40 Gluconic acid 60

Nanoparticles were synthesised as per Example 1.1.5., but gluconic acid (60mM) was used instead of tartaric and adipic acids. Nanoparticles synthesised with gluconic acid as per this example were characterised for copper phase distribution. During the synthetic process soluble copper converted to particulate copper oxo-hydroxide as pH increased. By pH 6, the particulate phase was mostly composed of nanoparticles (fraction greater than 80% of total copper). Nanoparticles synthesised with gluconic acid as per this example were characterised by Dynamic Light Scattering. When recovered at pH 8, these had hydrodynamic diameters between 1 and 10nm, with mean diameters between 2 and 4nm.

### 4.4 CuOH 20 Glutathione 20

Nanoparticles were synthesised as per Example 1.1.5., but glutathione (20mM) was used instead of tartaric and adipic acids. The initial concentration of $CuCl_2$ was also halved to 20mM. Nanoparticles synthesised with glutathione as per this example were characterised for copper phase distribution. During the synthetic process soluble copper converted to particulate copper oxo-hydroxide as pH increased. Between pH 3 and 4, the particulate phase was mostly composed of large agglomerates (approximately 70% of total copper). By pH 6, these micron-sized particles dispersed and the particulate copper became mostly composed of nanoparticles (fraction greater than 80% of total copper). Nanoparticles synthesised with glutathione as per Example N4 were characterised by Dynamic Light Scattering. When recovered at pH 8, these had hydrodynamic diameters between 1 and 5nm, with a mean diameter of approximately 2nm.

### 4.5 CuOH 40 Tart 20 Ad20 nanoparticles prepared with $Na_2CO_3$

Nanoparticles were synthesised as per Example 1.1.5, but $Na_2CO_3$ was used instead of NaOH. Nanoparticles synthesised using acid $Na_2CO_3$ as the titrant as per this example were characterised for copper phase distribution. During the synthetic process soluble copper converted to particulate copper oxo-hydroxide as pH increased. By pH 7, the particulate phase was mostly composed of nanoparticles (fraction greater than 90% of total copper). Nanoparticles synthesised using acid $Na_2CO_3$ as the titrant (as per Example N5) were characterised by Dynamic Light Scattering. When recovered at pH 8, these had hydrodynamic diameters between 1 and 8nm, with mean diameters between 2 and 4nm.

*4.6 Unmodified CuOH 40 (Comparative Example)*

The same synthetic methodology described in the Example 1.1.5. was followed, but in the absence of tartaric and adipic acid. During the synthetic process of unmodified copper hydroxides, most soluble copper converted to particulate between pH 4.3 and 5.2. Above this pH, the particulate phase was entirely composed of large micron-sized particles (fraction greater than 95% of total copper). The XRD spectrum of the resulting material was also obtained (Figure 11). The latter showed a crystalline pattern corresponding to paratacamite, a copper hydroxide of chemical formula $Cu_2(OH)_3Cl$ in which a chlorine atom was incorporated in the mineral structure (bottom).

*4.7 CuOH 40 Tart 20 nanoparticles*

Tartrate-modified copper oxo-hydroxide nanoparticles were synthesised as per Example 1.1.5., but in the absence of adipic acid. Nanoparticles synthesised in the absence of adipic acid as per this example were characterised by Dynamic Light Scattering. When recovered at pH 8, these had hydrodynamic diameters between 2 and 10nm, with mean diameters between 3 and 5nm.

*4.8 CuOH 2000 Tart 1000 nanoparticles*

Tartrate-modified copper oxo-hydroxide nanoparticles were synthesised as per Example 4.7, but at higher concentration (2.0 M copper and 1.0 M tartaric acid). The resulting material was a viscous slurry.

*4.9 Resuspension of CuOH 2000 Tart 1000 nanoparticles*

A slurry prepared as described in N9 was diluted to ~50mM in a 20mM adipic acid solution Cu and the pH adjusted to 8 with NaOH. Nanoparticles synthesised from a concentrated slurry as per this example were characterised by Dynamic Light Scattering. When recovered at pH 8, these had hydrodynamic diameters between 2 and 10nm, with mean diameters between 3 and 5nm.

*4.10 Removal of unbound ligands*

Free ligand and salts were removed through a process of ethanolic precipitation, in which a suspension of CuTartAd NPs (synthesised as per Example 1.1.5.) was mixed with ethanol on a volume ratio of 1:2 nanoparticle suspension: ethanol. Next, the agglomerated nanoparticles were span down at 1500rpm for 5 minutes and the supernatant (containing free ligands and salts) was discarded. The pellet, containing the nanoparticles, was resuspended to the original volume.

## *5. Activity Assay*

*5.1 Exposure of CuOH 40 Tart20 Ad20 nanoparticles to skin fibroblasts*

Human dermal fibroblast cells (cell line CCD-25SK) were incubated with CuTartAd NPs (0-200ppm Cu) in Minimum Essential Medium (containing L-glutamine and Earle's salts) supplemented with 5% heat inactivated Fetal Bovine Serum, 1% Penicillin-Streptomycin, 1% Fungizone and 3.8% bovine serum albumin, at 37 °C under a humidified 5% $CO_2$ atmosphere for 48 hours. $CuCl_2$ and $AgNO_3$ were also tested in parallel as positive controls. Percentage of cell confluence was determined experimentally using an IncuCyte Zoom and plotted overtime to determine the area under the curve (AUC) for each concentration tested. Cell proliferation was used as an indication for cell toxicity and was determined by normalising the AUC of cells exposed to the testing compounds against those of cells growing at normal rates (control).

Skin Fibroblasts cells were exposed to $CuCl_2$, $AgNO_3$ or ligand-modified copper nanoparticles (synthesised as per Example 1.1.5) for 48 hours. As shown in Figure 12, $CuCl_2$ and $AgNO_3$ caused a decrease in cell proliferation at lower concentrations (from 50mg/L and 10mg/L respectively) than with copper nanoparticles (from 100mg/L). In addition to reduced toxicity, copper oxo-hydroxide nanoparticles promoted cell growth (increased cell proliferation) at low concentrations (10 and 25 mg/L Cu) indicating a beneficial effect on wound healing.

**Claims**

**1.** An antibacterial composition for use in the treatment of wounds, wherein the composition comprises ligand-modified copper oxo-hydroxide nanoparticles and a pharmaceutically acceptable carrier, wherein the copper oxo-hydroxide nanoparticles have a structure in which the one or more ligands are non-stoichiometrically substituted for the oxo or hydroxy groups, wherein the one or more ligands comprise a carboxylic acid ligand, or an ionised form thereof and the copper is present in pharmaceutical formulation as free copper ions ($Cu^{2+}$).

2. An antibacterial composition for use in the treatment or prevention of a microbial infection, wherein the composition comprises ligand-modified copper oxo-hydroxide nanoparticles and a pharmaceutically acceptable carrier, wherein the copper oxo-hydroxide nanoparticles have a structure in which the one or more ligands are non-stoichiometrically substituted for the oxo or hydroxy groups, wherein the one or more ligands comprise a carboxylic acid ligand, or an ionised form thereof and the copper is present in pharmaceutical formulation as free copper ions ($Cu^{2+}$).

3. The antibacterial composition comprising copper oxo-hydroxide nanoparticles for use in the treatment or prevention according to claim 1 or claim 2, wherein the composition is for treating a human or animal subject.

4. The antibacterial composition comprising copper oxo-hydroxide nanoparticles for use in the treatment or prevention according to claim 2 or claim 3, wherein the microbial infection is a bacterial infection or a fungal infection.

5. The antibacterial composition comprising copper oxo-hydroxide nanoparticles for use in the treatment or prevention according to any one of claims 2 to 4, wherein the infection is caused by a gram-negative or a gram-positive bacterium.

6. The antibacterial composition comprising copper oxo-hydroxide nanoparticles for use in the treatment or prevention according to any one of claims 2 to 5, wherein the bacterial infection is caused by an *Escherichia sp., such as E. coli,* a *Staphylococcus sp., such as S. epidermis, S. aureus* or meticillin-resistant staphylococcus aureus ("MRSA"), a *Bacillus sp.,* such as *B. subtilis,* a *Pseudomonas sp., such as P. aeruginosa,* a *Vibrio sp.,* such as *V. fisheri,* a *Streptococcus sp., such as S. pyrogenes* and *S. pneumoniae,* a *Klebsiella sp.,* a *Micrococcus sp., such as M. luteus,* a *Clostridium sp.* such as *C. difficile,* an *Acinetobacter sp.* such as *A. baumannii,* a *Mycobacterium sp.,* such as *M. tuberculosis* or a *Salmonella sp.* a *Chlamydia sp.* or a fungal species such as a *Candida sp., such as C. albicans.*

7. The antibacterial composition comprising copper oxo-hydroxide nanoparticles for use in the treatment or prevention according to any one of claims 1 to 6, wherein the composition is for veterinary administration.

8. The antibacterial composition comprising copper oxo-hydroxide nanoparticles for use in the treatment or prevention according to any one of claims 1 to 7, wherein the treatment of prevention comprises coating or treating an article with an antibacterial composition.

9. The antibacterial composition comprising copper oxo-hydroxide nanoparticles for use in the treatment or prevention according to claim 8, wherein the article is an implantable medical device or a coated substrate, such as a non-woven fabric substrate.

10. The antibacterial composition comprising copper oxo-hydroxide nanoparticles for use in the treatment or prevention according to any one of claims 1 to 7, wherein the composition is formulated for topical administration.

11. The antibacterial composition comprising copper oxo-hydroxide nanoparticles for use in the treatment or prevention according to any one of claims 1 to 9, wherein the composition is for the treatment of a skin disorder or a disorder of mucous membranes.

12. The antibacterial composition comprising copper oxo-hydroxide nanoparticles for use in the treatment or prevention according to any one of the preceding claims, wherein the material has a polymeric structure in which the ligands are distributed within the solid phase structure of the copper oxo-hydroxide.

13. The antibacterial composition comprising copper oxo-hydroxide nanoparticles for use in the treatment or prevention according to any one of the preceding claims, wherein the ligand-modified copper oxo-hydroxide nanoparticles have one or more reproducible physicochemical properties, wherein the one or more reproducible physicochemical properties are selected from dissolution profile, release of soluble copper as a percentage of total copper present in the composition and/or antibacterial activity as determined in a growth inhibition assay.

14. The antibacterial composition comprising copper oxo-hydroxide nanoparticles for use in the treatment or prevention according to any one of the preceding claims, wherein the carboxylic acid ligand is a linear or cyclic mono or dicarboxylic acid ligand, optionally wherein the carboxylic acid ligand or the ionised form thereof is tartaric acid or tartarate, gluconic acid or gluconate, adipic acid or adipate, succinic acid or succinate, malic acid or malinate, glutaric acid or glutarate, pimelic acid or pimelate and/or glutathione, or wherein the carboxylic acid ligand is an amino acid or a sugar acid.

15. The antibacterial composition comprising copper oxo-hydroxide nanoparticles for use in the treatment or prevention according to any one of the preceding claims, wherein:

(a) the carboxylic acid ligand or the ionised form thereof is tartaric acid or tartarate or gluconic acid or gluconate or glutathione; or
(b) the carboxylic acid ligand or the ionised form thereof is tartaric acid or tartarate in combination with adipic acid or adipate.

16. The antibacterial composition comprising copper oxo-hydroxide nanoparticles for use in the treatment or prevention according to any one of the preceding claims, wherein:

(a) the nanoparticles in the composition have demonstrable M-L bonding as determined using infrared spectroscopy; and/or
(b) the nanoparticles in the composition are substantially amorphous as determined by XRD.

17. The antibacterial composition comprising copper oxo-hydroxide nanoparticles for use in the treatment or prevention according to any one of the preceding claims, wherein the nanoparticles have a mean diameter between 1 and 100 nm, and optionally wherein the nanoparticles have a mean diameter between 1 and 10 nm.

18. The antibacterial composition comprising copper oxo-hydroxide nanoparticles for use in the treatment or prevention according to any one of the preceding claims, wherein the composition is formulated at a pH between 6.0 and 8.0.

19. The antibacterial composition comprising copper oxo-hydroxide nanoparticles for use in the treatment or prevention according to any one of the preceding claims, wherein the composition further comprises a matrix in which the nanoparticles are formulated.

20. The antibacterial composition comprising copper oxo-hydroxide nanoparticles for use in the treatment or prevention according to claim 19, wherein the matrix comprises hydroxyethylcellulose or PEG.

21. The antibacterial composition comprising copper oxo-hydroxide nanoparticles for use in the treatment or prevention according to any one of the preceding claims, wherein the composition is for topical delivery, vaginal delivery, nasal delivery, rectal delivery or oral delivery.

22. A process for producing a copper oxo-hydroxide nanoparticle pharmaceutical composition according to any one of claims 1 to 21, the process comprising:

(a) mixing the solution comprising $Cu^{2+}$ and a carboxylic acid ligand, and optionally one or more further ligands or reaction components, in a reaction medium at a first pH(A) at which the components are soluble;
(b) changing the pH(A) to a second pH(B) to cause a solid precipitate or a colloid of the copper oxo-hydroxide nanoparticle composition to be formed;
(c) separating, and optionally drying and/or formulating, the copper oxo-hydroxide nanoparticle composition produced in step (b).

**Patentansprüche**

1. Antibakterielle Zusammensetzung zur Verwendung bei der Behandlung von Wunden, wobei die Zusammensetzung Liganden-modifizierte Kupferoxohydroxid-Nanopartikel und einen pharmazeutisch annehmbaren Träger umfasst, wobei die Kupferoxohydroxid-Nanopartikel eine Struktur aufweisen, in der der eine oder die mehreren Ligand(en) nicht stöchiometrisch für die Oxo- oder die Hydroxygruppen substituiert ist/sind, wobei der eine oder die mehreren Ligand(en) einen Carbonsäureliganden oder eine ionisierte Form davon umfasst/umfassen und Kupfer in der pharmazeutischen Formulierung als freie Kupferionen ($Cu^{2+}$) vorliegt.

2. Antibakterielle Zusammensetzung zur Verwendung bei der Behandlung oder Prävention einer mikrobiellen Infektion, wobei die Zusammensetzung Liganden-modifizierte Kupferoxohydroxid-Nanopartikel und einen pharmazeutisch annehmbaren Träger umfasst, wobei die Kupferoxohydroxid-Nanopartikel eine Struktur aufweisen, in der der eine oder die mehreren Ligand(en) nicht stöchiometrisch für die Oxo- oder die Hydroxygruppen substituiert ist/sind, wobei der eine oder die mehreren Ligand(en) einen Carbonsäureliganden oder eine ionisierte Form davon umfasst/umfassen und Kupfer in der pharmazeutischen Formulierung als freie Kupferionen ($Cu^{2+}$) vorliegt.

3. Antibakterielle Zusammensetzung, umfassend Kupferoxohydroxid-Nanopartikel, zur Verwendung bei der Behandlung oder Prävention nach Anspruch 1 oder 2, wobei die Zusammensetzung zur Behandlung eines menschlichen oder eines tierischen Individuums vorgesehen ist.

4. Antibakterielle Zusammensetzung, umfassend Kupferoxohydroxid-Nanopartikel, zur Verwendung bei der Behandlung oder Prävention nach Anspruch 2 oder 3, wobei die mikrobielle Infektion eine bakterielle Infektion oder eine Pilzinfektion ist.

5. Antibakterielle Zusammensetzung, umfassend Kupferoxohydroxid-Nanopartikel, zur Verwendung bei der Behandlung oder Prävention nach einem der Ansprüche 2 bis 4, wobei die Infektion durch ein Gram-negatives oder ein Gram-positives Bakterium verursacht wird.

6. Antibakterielle Zusammensetzung, umfassend Kupferoxohydroxid-Nanopartikel, zur Verwendung bei der Behandlung oder Prävention nach einem der Ansprüche 2 bis 5, wobei die bakterielle Infektion durch eine *Escherichia sp.,* wie z.B. *E. coli,* eine *Staphylococcus sp.,* wie z.B. *S. epidermis, S. aureus* oder Meticillin-resistenten Staphylococcus aureus ("MRSA"), eine *Bacillus sp.,* wie z.B. *B. subtilis,* eine *Pseudomonas sp.,* wie z.B. *P. aeruginosa,* eine *Vibrio sp.,* wie z.B. *V. fisheri,* eine *Streptococcus sp.,* wie z.B. *S. pyrogenes* und *S. pneumoniae,* eine *Klebsiella sp.,* eine *Micrococcus sp.,* wie z.B. *M. luteus,* eine *Clostridium sp.,* wie z.B. *C. difficile,* eine *Acinetobacter sp.,* wie z.B. *A. baumannii,* eine *Mycobacterium sp.,* wie z.B. *M. tuberculosis,* oder eine *Salmonella sp.,* eine *Chlamydia sp.* oder eine Pilzart, wie z.B. eine *Candida sp.,* wie z.B. *C. albicans,* verursacht wird.

7. Antibakterielle Zusammensetzung, umfassend Kupferoxohydroxid-Nanopartikel, zur Verwendung bei der Behandlung oder Prävention nach einem der Ansprüche 1 bis 6, wobei die Zusammensetzung für Veterinärverabreichung vorgesehen ist.

8. Antibakterielle Zusammensetzung, umfassend Kupferoxohydroxid-Nanopartikel, zur Verwendung bei der Behandlung oder Prävention nach einem der Ansprüche 1 bis 7, wobei die Behandlung oder Prävention das Beschichten oder Versetzen eines Artikels mit einer antibakteriellen Zusammensetzung umfasst.

9. Antibakterielle Zusammensetzung, umfassend Kupferoxohydroxid-Nanopartikel, zur Verwendung bei der Behandlung oder Prävention nach Anspruch 8, wobei der Artikel eine implantierbare medizinische Vorrichtung oder ein beschichtetes Substrat, wie z. B. ein Vliesstoffsubstrat, ist.

10. Antibakterielle Zusammensetzung, umfassend Kupferoxohydroxid-Nanopartikel, zur Verwendung bei der Behandlung oder Prävention nach einem der Ansprüche 1 bis 7, wobei die Zusammensetzung für topische Verabreichung formuliert ist.

11. Antibakterielle Zusammensetzung, umfassend Kupferoxohydroxid-Nanopartikel, zur Verwendung bei der Behandlung oder Prävention nach einem der Ansprüche 1 bis 9, wobei die Zusammensetzung zur Behandlung einer Hautstörung oder einer Störung der Schleimhautmembranen vorgesehen ist.

12. Antibakterielle Zusammensetzung, umfassend Kupferoxohydroxid-Nanopartikel, zur Verwendung bei der Behandlung oder Prävention nach einem der vorangegangenen Ansprüche, wobei das Material eine Polymerstruktur aufweist, in der die Liganden in der Festphasenstruktur des Kupferoxohydroxids verteilt sind.

13. Antibakterielle Zusammensetzung, umfassend Kupferoxohydroxid-Nanopartikel, zur Verwendung bei der Behandlung oder Prävention nach einem der vorangegangenen Ansprüche, wobei die Liganden-modifizierten Kupferoxohydroxid-Nanopartikel eine oder mehrere reproduzierbare physikochemische Eigenschaft(en) aufweisen, wobei die eine oder mehreren reproduzierbare(n) physiochemische(n) Eigenschaft(en) aus Auflösungsprofil, Freisetzung von löslichem Kupfer als Prozentsatz des gesamten Kupfers, das in der Zusammensetzung vorhanden ist, und/oder antibakterielle Aktivität wie im Wachstumsinhibitionstest bestimmt ausgewählt ist/sind.

**14.** Antibakterielle Zusammensetzung, umfassend Kupferoxohydroxid-Nanopartikel, zur Verwendung bei der Behandlung oder Prävention nach einem der vorangegangenen Ansprüche, wobei der Carbonsäureligand ein linearer oder zyklischer Mono- oder Dicarbonsäureligand ist, wobei der Carbonsäureligand oder die ionisierte Form davon gegebenenfalls Weinsäure oder Tartrat, Gluconsäure oder Gluconat, Adipinsäure oder Adipat, Bernsteinsäure oder Succinat, Äpfelsäure oder Malinat, Glutarsäure oder Glutarat, Pimelinsäure oder Pimelat und/oder Glutathion ist oder wobei der Carbonsäureligand eine Aminosäure oder eine Zuckersäure ist.

**15.** Antibakterielle Zusammensetzung, umfassend Kupferoxohydroxid-Nanopartikel, zur Verwendung bei der Behandlung oder Prävention nach einem der vorangegangenen Ansprüche, wobei:

(a) der Carbonsäureligand oder die ionisierte Form davon Weinsäure oder Tartrat oder Gluconsäure oder Gluconat oder Glutathion ist; oder
(b) der Carbonsäureligand oder die ionisierte Form davon Weinsäure oder Tartrat in Kombination mit Adipinsäure oder Adipat ist.

**16.** Antibakterielle Zusammensetzung, umfassend Kupferoxohydroxid-Nanopartikel, zur Verwendung bei der Behandlung oder Prävention nach einem der vorangegangenen Ansprüche, wobei:

(a) die Nanopartikel in der Zusammensetzung nachweisbare M-L-Bindung aufweisen, wie mittels Infrarotspektroskopie bestimmt wird; und/oder
(b) die Nanopartikel in der Zusammensetzung im Wesentlichen amorph sind, wie mittels XRD bestimmt wird.

**17.** Antibakterielle Zusammensetzung, umfassend Kupferoxohydroxid-Nanopartikel, zur Verwendung bei der Behandlung oder Prävention nach einem der vorangegangenen Ansprüche, wobei die Nanopartikel einen mittleren Durchmesser zwischen 1 und 100 nm aufweisen und wobei die Nanopartikel gegebenenfalls einen mittleren Durchmesser zwischen 1 und 10 nm aufweisen.

**18.** Antibakterielle Zusammensetzung, umfassend Kupferoxohydroxid-Nanopartikel, zur Verwendung bei der Behandlung oder Prävention nach einem der vorangegangenen Ansprüche, wobei die Zusammensetzung bei einem pH zwischen 6,0 und 8,0 formuliert ist.

**19.** Antibakterielle Zusammensetzung, umfassend Kupferoxohydroxid-Nanopartikel, zur Verwendung bei der Behandlung oder Prävention nach einem der vorangegangenen Ansprüche, wobei die Zusammensetzung weiters eine Matrix umfasst, in der die Nanopartikel formuliert sind.

**20.** Antibakterielle Zusammensetzung, umfassend Kupferoxohydroxid-Nanopartikel, zur Verwendung bei der Behandlung oder Prävention nach Anspruch 19, wobei die Matrix Hydroxyethylcellulose oder PEG umfasst.

**21.** Antibakterielle Zusammensetzung, umfassend Kupferoxohydroxid-Nanopartikel, zur Verwendung bei der Behandlung oder Prävention nach einem der vorangegangenen Ansprüche, wobei die Zusammensetzung für topische Zufuhr, vaginale Zufuhr, nasale Zufuhr, rektale Zufuhr oder orale Zufuhr vorgesehen ist.

**22.** Verfahren zur Herstellung einer pharmazeutischen Kupferoxohydroxid-Nanopartikel-Zusammensetzung nach einem der Ansprüche 1 bis 21, wobei das Verfahren Folgendes umfasst:

(a) das Vermischen der Lösung, die $Cu^{2+}$ und einen Carbonsäureliganden und gegebenenfalls einen oder mehrere weitere Liganden oder Reaktionskomponenten umfasst, in einem Reaktionsmedium bei einem ersten pH(A), bei dem die Komponenten löslich sind;
(b) das Verändern des pH(A) auf einen zweiten pH(B), um das Ausbilden eines festen Niederschlags oder eines Kolloids der Kupferoxohydroxid-Nanopartikel-Zusammensetzung zu verursachen;
(c) das Trennen und gegebenenfalls Trocknen und/oder Formulieren der in Schritt (b) erzeugten Kupferoxohydroxid-Nanopartikel-Zusammensetzung.

**Revendications**

**1.** Composition antibactérienne pour une utilisation dans le traitement de blessures, dans laquelle la composition comprend des nanoparticules d'oxo-hydroxyde de cuivre modifiées par un ligand et un véhicule pharmaceutiquement acceptable, dans laquelle les nanoparticules d'oxo-hydroxyde de cuivre ont une structure dans laquelle le ou les ligands sont substitués de manière non stœchiométrique pour les groupes oxo ou hydroxy, dans laquelle le ou les ligands comprennent un ligand acide carboxylique, ou une forme ionisée de celui-ci et le cuivre est présent dans la formulation pharmaceutique sous forme d'ions cuivre libres ($Cu^{2+}$).

2. Composition antibactérienne pour une utilisation dans le traitement ou la prévention d'une infection microbienne, dans laquelle la composition comprend des nanoparticules d'oxo-hydroxyde de cuivre modifiées par un ligand et un véhicule pharmaceutiquement acceptable, dans laquelle les nanoparticules d'oxo-hydroxyde de cuivre ont une structure dans laquelle le ou les ligands sont substitués de manière non stœchiométrique pour les groupes oxo ou hydroxy, dans laquelle le ou les ligands comprennent un ligand acide carboxylique, ou une forme ionisée de celui-ci et le cuivre est présent dans la formulation pharmaceutique sous forme d'ions cuivre libres ($Cu^{2+}$).

3. Composition antibactérienne comprenant des nanoparticules d'oxo-hydroxyde de cuivre pour une utilisation dans le traitement ou la prévention selon la revendication 1 ou la revendication 2, dans laquelle la composition est destinée au traitement d'un sujet humain ou animal.

4. Composition antibactérienne comprenant des nanoparticules d'oxo-hydroxyde de cuivre pour une utilisation dans le traitement ou la prévention selon la revendication 2 ou la revendication 3, dans laquelle l'infection microbienne est une infection bactérienne ou une infection fongique.

5. Composition antibactérienne comprenant des nanoparticules d'oxo-hydroxyde de cuivre pour une utilisation dans le traitement ou la prévention selon l'une quelconque des revendications 2 à 4, dans laquelle l'infection est provoquée par une bactérie à gram négatif ou à gram positif.

6. Composition antibactérienne comprenant des nanoparticules d'oxo-hydroxyde de cuivre pour une utilisation dans le traitement ou la prévention selon l'une quelconque des revendications 2 à 5, dans laquelle l'infection bactérienne est provoquée par un *Escherichia sp.,* comme *E. coli,* un *Staphylococcus sp.,* comme *S. epidermis, S. aureus* ou le staphylococcus aureus résistant à la méticilline (« SARM »), un *Bacillus sp.,* comme *B. subtilis,* un *Pseudomonas sp.,* comme *P. aeruginosa,* un *Vibrio sp.,* comme *V. fisheri,* un *Streptococcus sp.,* comme *S. pyrogenes* et *S. pneumoniae,* un *Klebsiella sp.,* un *Micrococcus sp.* comme *M. luteus,* un *Clostridium sp.* comme *C. difficile,* un *Acinetobacter sp.* comme A. *baumannii,* un *Mycobacterium sp.,* comme *M. tuberculosis* ou un *Salmonella sp.,* un *Chlamydia sp.* ou une espèce fongique comme un *Candida sp.,* comme *C. albicans.*

7. Composition antibactérienne comprenant des nanoparticules d'oxo-hydroxyde de cuivre pour une utilisation dans le traitement ou la prévention selon l'une quelconque des revendications 1 à 6, dans laquelle la composition est destinée à l'administration vétérinaire.

8. Composition antibactérienne comprenant des nanoparticules d'oxo-hydroxyde de cuivre pour une utilisation dans le traitement ou la prévention selon l'une quelconque des revendications 1 à 7, dans laquelle le traitement de la prévention comprend le revêtement ou le traitement d'un article avec une composition bactérienne.

9. Composition antibactérienne comprenant des nanoparticules d'oxo-hydroxyde de cuivre pour une utilisation dans le traitement ou la prévention selon la revendication 8, dans laquelle l'article est un dispositif médical implantable ou un substrat enduit, comme un substrat de tissu non tissé.

10. Composition antibactérienne comprenant des nanoparticules d'oxo-hydroxyde de cuivre pour une utilisation dans le traitement ou la prévention selon l'une quelconque des revendications 1 à 7, dans laquelle la composition est formulée pour une administration topique.

11. Composition antibactérienne comprenant des nanoparticules d'oxo-hydroxyde de cuivre pour une utilisation dans le traitement ou la prévention selon l'une quelconque des revendications 1 à 9, dans laquelle la composition est destinée au traitement d'une maladie de la peau ou d'un trouble des muqueuses.

12. Composition antibactérienne comprenant des nanoparticules d'oxo-hydroxyde de cuivre pour une utilisation dans le traitement ou la prévention selon l'une quelconque des revendications précédentes, dans laquelle le matériau a une structure polymère dans laquelle les ligands sont distribués au sein de la structure de phase solide de l'oxo-hydroxyde de cuivre.

13. Composition antibactérienne comprenant des nanoparticules d'oxo-hydroxyde de cuivre pour une utilisation dans le traitement ou la prévention selon l'une quelconque des revendications précédentes, dans laquelle les nanoparticules d'oxo-hydroxyde de cuivre modifiée par un ligand ont une ou plusieurs propriétés physicochimiques reproductibles, dans laquelle la ou les propriétés physicochimiques reproductibles sont choisies parmi un profil de dissolution, la libération de cuivre soluble en tant que pourcentage de cuivre total présent dans la composition et/ou une activité antibactérienne telle que déterminée dans un dosage d'inhibition de croissance.

14. Composition antibactérienne comprenant des nanoparticules d'oxo-hydroxyde de cuivre pour une utilisation dans le traitement ou la prévention selon l'une quelconque des revendications précédentes, dans laquelle le ligand acide carboxylique est un ligand acide mono ou dicarboxylique linéaire ou cyclique, éventuellement dans laquelle le ligand acide carboxylique ou la forme ionisée de celui-ci est l'acide tartrique ou le tartrate, l'acide gluconique ou le gluconate, l'acide adipique ou l'adipate, l'acide succinique ou le succinate, l'acide malique ou le malinate, l'acide glutarique ou le glutarate, l'acide pimélique ou le pimélate et/ou le glutathion, ou dans laquelle le ligand acide carboxylique est un acide aminé ou un acide de sucre.

15. Composition antibactérienne comprenant des nanoparticules d'oxo-hydroxyde de cuivre pour une utilisation dans le traitement ou la prévention selon l'une quelconque des revendications précédentes, dans laquelle :

(a) le ligand acide carboxylique ou la forme ionisée de celui-ci est l'acide tartrique ou le tartrate ou l'acide gluconique ou le gluconate ou le glutation ; ou
(b) le ligand acide carboxylique ou la forme ionisée de celui-ci est l'acide tartrique ou le tartrate en combinaison avec l'acide adipique ou l'adipate.

16. Composition antibactérienne comprenant des nanoparticules d'oxo-hydroxyde de cuivre pour une utilisation dans le traitement ou la prévention selon l'une quelconque des revendications précédentes, dans laquelle :

(a) les nanoparticules dans la composition ont une liaison M-L démontrable comme déterminé en utilisant une spectroscopie infrarouge ; et/ou
(b) les nanoparticules dans la composition sont sensiblement amorphes comme déterminé par XRD.

17. Composition antibactérienne comprenant des nanoparticules d'oxo-hydroxyde de cuivre pour une utilisation dans le traitement ou la prévention selon l'une quelconque des revendications précédentes, dans laquelle les nanoparticules ont un diamètre moyen entre 1 et 100 nm, et éventuellement dans laquelle les nanoparticules ont un diamètre moyen entre 1 et 10 nm.

18. Composition antibactérienne comprenant des nanoparticules d'oxo-hydroxyde de cuivre pour une utilisation dans le traitement ou la prévention selon l'une quelconque des revendications précédentes, dans laquelle la composition est formulée à un pH entre 6,0 et 8,0.

19. Composition antibactérienne comprenant des nanoparticules d'oxo-hydroxyde de cuivre pour une utilisation dans le traitement ou la prévention selon l'une quelconque des revendications précédentes, dans laquelle la composition comprend en outre une matrice dans laquelle les nanoparticules sont formulées.

20. Composition antibactérienne comprenant des nanoparticules d'oxo-hydroxyde de cuivre pour une utilisation dans le traitement ou la prévention selon la revendication 19, dans laquelle la matrice comprend de l'hydroxyéthylcellulose ou un PEG.

21. Composition antibactérienne comprenant des nanoparticules d'oxo-hydroxyde de cuivre pour une utilisation dans le traitement ou la prévention selon l'une quelconque des revendications précédentes, dans laquelle la composition est pour une administration topique, une administration vaginale, une administration nasale, une administration rectale ou une administration orale.

22. Procédé de production d'une composition pharmaceutique de nanoparticules d'oxo-hydroxyde de cuivre selon l'une quelconque des revendications 1 à 21, le procédé comprenant :

(a) le mélange de la solution comprenant du $Cu^{2+}$ et un ligand acide carboxylique, et éventuellement un ou plusieurs autres ligands ou composants réactionnels, dans un milieu de réaction à un premier pH (A) auquel les composants sont solubles ;

(b) le changement du pH (A) en un second pH (B) pour provoquer la formation d'un précipité solide ou d'un colloïde de la composition de nanoparticules d'oxo-hydroxyde de cuivre ;

(c) la séparation, et éventuellement le séchage et/ou la formulation, de la composition de nanoparticules d'oxo-hydroxyde de cuivre produite à l'étape (b).

Figure 1

Figure 2

Figure 3

Figure 4

Figure 5

Figure 6

Figure 7

Figure 8

Figure 9

Figure 10

Figure 11

Figure 12

## REFERENCES CITED IN THE DESCRIPTION

**Patent documents cited in the description**

- GB 1600449 A
- WO 2008096130 A
- WO 2010015827 A
- WO 2012101407 A
- DE 20205014332
- US 20080147019 A

**Non-patent literature cited in the description**

- Remington's Pharmaceutical Sciences. Lippincott, Williams & Wilkins, 2000
- **SCHLOSSMAN et al.** *The Chemistry and Manufacture of Cosmetics,* 1998